(19) Europäisches Patentamt
European Patent Office
Office européen des brevets

(11) EP 4 488 351 A1

(12) EUROPEAN PATENT APPLICATION

(43) Date of publication:
**08.01.2025 Bulletin 2025/02**

(21) Application number: **23183075.3**

(22) Date of filing: **03.07.2023**

(51) International Patent Classification (IPC):
***C11D 3/38*** *(2006.01)* ***C11D 11/00*** *(2006.01)*
***C11D 1/02*** *(2006.01)* ***C11D 3/386*** *(2006.01)*
***C07K 14/195*** *(2006.01)*

(52) Cooperative Patent Classification (CPC):
**C11D 3/386; C07K 14/195; C11D 1/02; C11D 3/38;**
C11D 2111/12; C11D 2111/14

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **The Procter & Gamble Company**
**Cincinnati, OH 45202 (US)**

(72) Inventors:
- **AZZI, Adam Amin**
  **Newcastle upon Tyne, NE12 9 TS (GB)**
- **LANT, Neil Joseph**
  **Newcastle upon Tyne, NE12 9TS (GB)**
- **MORALES GARCIA, Ana L.**
  **Newcastle-upon-Tyne, NE12 9TS (GB)**
- **YAU, Hamish Chun Lam**
  **Newcastle upon Tyne, NE12 9TS (GB)**
- **BLACK, Gary William**
  **Newcastle upon Tyne, NE1 8ST (GB)**
- **MUNOZ MUNOZ, José Luis**
  **Newcastle upon Tyne, NE1 8ST (GB)**
- **WILLATS, William George Tycho**
  **Newcastle upon Tyne, NE1 7RU (GB)**

(74) Representative: **P&G Patent Belgium UK**
**N.V. Procter & Gamble Services Company S.A.**
**Temselaan 100**
**1853 Strombeek-Bever (BE)**

Remarks:
The complete document including Reference Table(s) and the Sequence Listing(s) can be downloaded from the EPO website

(54) **COMPOSITIONS CONTAINING A PORPHYRIN BINDING PROTEIN**

(57) The invention relates to a treatment composition comprising a porphyrin binding protein.

EP 4 488 351 A1

## Description

REFERENCE TO A SEQUENCE LISTING

This application contains a Sequence Listing in computer readable form. The computer readable form is incorporated herein by reference.

FIELD OF THE INVENTION

The present invention relates to compositions comprising a porphyrin binding protein. The invention also relates to methods of treatment using the composition of the invention and the use of the porphyrin binding protein in treatment compositions.

BACKGROUND OF THE INVENTION

In cleaning and treatment applications, particularly for dishwashing (automatic and manual), hard surface cleaning, laundry, oral care, surgical device cleaning and pharmaceutical applications, stain, soil, and malodour removal can present permanent unresolved problems. There are many cleaning technologies aimed at mitigating such problems however, it is a constant challenge to provide improved efficacy and especially in an environmentally favourable manner. In automatic dishwashing and laundry washing machines these problems are compounded by the increased use of low wash temperatures (e.g., cold water) and shorter washing cycles which can reduce stain/soil/malodor removal efficacy of cleaning compositions and exacerbate problems of redeposition of soil onto surfaces during the washing process.

Enzymes are routinely used in cleaning and treatment compositions including laundry detergents, automatic dishwashing detergents, toothpastes and pharmaceuticals, where these proteins catalyse the breakdown of substrates with beneficial effects such as improved soil or malodor removal. There is a constant search for new approaches to assist with such processes which operate by different mechanisms and are environmentally-friendly. Thus, it is an object of the present invention to provide a treatment composition which exhibits soil and/or stain and/or malodor removal. 'Soil' is defined as undesired contaminants targeted for removal in any cleaning or treatment composition.

SUMMARY OF THE INVENTION

According to the first aspect of the invention, there is provided a treatment composition. The composition comprises a porphyrin binding protein. The composition also comprises at least one adjunct component. The adjunct component is preferably selected from a surfactant, a builder, a bleach component, a polymer, a dispersing agent, and an enzyme. Preferably, the composition comprises an enzyme. Preferably, the composition comprises a surfactant. Preferably, the composition comprises an enzyme and a surfactant. The composition can be any treatment composition, toothpaste, mouthwash, hard surface cleaning compositions, dishwashing cleaning compositions, laundry cleaning compositions, etc. Especially useful compositions for use herein are laundry and automatic dishwashing compositions. The composition can be an alkaline composition. By "alkaline composition" is herein understood a composition having a pH above 7, preferably from 7.5 to 11, more preferably from 8 to 11, as measured in a 10% by weight aqueous composition at 25°C.

According to the second aspect of the invention, there is provided a method of treating a surface using the composition of the invention. The surface can be a hard surface, such as a dishware surface or a soft surface, such as a fabric. In the case of a method of treating a fabric, the method comprises contacting a fabric with an aqueous wash liquor comprising the composition of the invention. Preferably the aqueous wash liquor comprises anionic surfactant in an amount from 0.05g/l to 5g/l, preferably from 0.01g/l to 3g/l. The fabric is typically contacted with the aqueous wash liquor at a temperature of 60°C or less, preferably at a temperature of 40°C or less or 35°C or less, most preferably at a temperature of 30°C or less or even 25°C or less; and (iii) optionally rinsing the surface. The compositions and methods herein are particularly useful for treating any dishware or fabric surface. Fabric surfaces include synthetic or natural, including cotton, wool, silk, polyester, nylon, elastane or mixed fabric, such as polycotton.

The composition and method described herein may also promote biofilm-disrupting effects.

According to the third aspect of the invention, there is provided the use of a porphyrin binding protein to provide cleaning. The treatment composition comprising a porphyrin binding protein can provide stain, soil and/or malodour removal, in particular, the use of a porphyrin binding protein in a cleaning composition can provide good removal of organic stains such as blood stains and grass stains, preferably from fabrics. The composition of the invention also provides good cleaning of dingy fabrics.

The elements of the composition of the invention described herein apply *mutatis mutandis* to the use and method aspects of the invention.

DETAILED DESCRIPTION OF THE INVENTION

Definitions

For the purpose of the present invention, a "porphyrin binding protein" is a protein capable of binding porphyrin. Proteins that bind porphyrin exhibits a shift in $\lambda_{max}$ profile between 390-410nm, compared to a similar sample containing porphyrin and nil porphyrin binding protein.

Sequence Identity: The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labelled "longest identity" (obtained using the -nobrief option) is used as the percent identity and is calculated as follows:

$$(\text{Identical Residues x } 100)/ (\text{Length of Alignment} - \text{Total Number of Gaps in Alignment})$$

All percentages, ratios and proportions used herein are by weight percent of the composition, unless otherwise specified. All average values are calculated "by weight" of the composition, unless otherwise expressly indicated.

All measurements are performed at 25°C unless otherwise specified.

3-dimensional (3D) structure identity: The relatedness between two 3-dimensional structures derived from atomic co-ordinates is described by the parameter "3D structure identity". For purposes of the present invention, the degree of 3-dimensional structure identity between two 3D structures is determined using an algorithm implanted in a Fortran-77 program called Dali (Distance matrix alignment algorithm), as referenced, and described in: Holm L. Using Dali for Protein Structure Comparison. Methods Mol Biol. 2020; 2112:29-42, and references cited therein.

Atomic co-ordinates for input structures are preferably downloaded from the Protein Data Bank (PDB, RCSB.org). If no PDB entry is available then atomic co-ordinates are computationally modelled and downloaded via EMBL-EBI hosted Alphafold 3D protein structure database (Senior, A.W., Evans, R., Jumper, J. et al. Improved protein structure prediction using potentials from deep learning. Nature 577, 706-710 (2020)) in a suitable format, i.e., Crystallographic info file format. The input structure must have complete backbone atoms (C, CA, N, O). Downloaded atomic co-ordinate files are inputted into the Dali server (available at http://ekhidna.biocenter.helsinki.fi/dali_server) for a pairwise comparison of protein structures, preferably running DaliLite V.3. or newer. (Holm L, Kääriäinen S, Rosenström P, Schenkel A. Searching protein structure databases with DaliLite v.3. Bioinformatics. 2008 Dec 1;24(23):2780-1).

Algorithm (DALI) for optimal pairwise alignment of protein structures. The three-dimensional co-ordinates of each protein are used to calculate residue-residue (C alpha-C alpha) distance matrices. The distance matrices are first decomposed into elementary contact patterns, e.g., hexapeptide-hexapeptide submatrices. Then, similar contact patterns in the two matrices are paired and combined into larger consistent sets of pairs. An iterative Monte Carlo optimization procedure is used to optimize a similarity score defined in terms of equivalent intramolecular distances (Metropolis, N., Rosenbluth, A. W., Rosenbluth, M. N., Teller, A. H. & Teller, E. (1953) Equation of state calculations by fast computing machines. J. Chem. Phys. 21, 1087-1092.). Several alignments are optimized in parallel, leading to simultaneous detection of the best, second-best and so on solutions. The method allows sequence gaps of any length, reversal of chain direction and free topological connectivity of aligned segments.

Pairwise comparison through implementation of four structure alignment algorithms

1. Soap algorithm described in 'Falicov A, Cohen FE (1996) A surface of minimum area metric for the structural comparison of proteins. J Mol Biol 258:871-892', is used to align structures with few secondary structure elements. Soap minimizes a "soap film" metric between two $C\alpha$ traces superimposed in 3D space. The minimal surface area between the virtual backbones of two proteins is determined numerically using an iterative triangulation strategy. The first protein is then rotated and translated in space until the smallest minimal surface is obtained. Such a technique yields the optimal structural superposition between two protein segments.
2. Wolf algorithm described in Holm L. Using Dali for Protein Structure Comparison. Methods Mol Biol. 2020; 2112:29-42. Three points taken from an ordered pair of secondary structure elements (SSE) define an internal coordinate frame. Here, the midpoint of the first SSE is the origin, the vector representing the first SSE aligns with the y axis, and the midpoint of the second SSE is in the positive z-y half-plane. Each database structure is presented in poses defined by all possible frames of SSE pairs. Testing all frames of the query structure allows counting the number

of matching SSEs at nearby positions in all possible poses. The result is a ranked list of database structures which can be used as a filter in a database search.

3. Parsi alignment algorithm described in Holm L, Sander C (1996) Mapping the protein universe. Science 273:595-602. The algorithm delivers an exact solution to the subproblem of ungapped alignment of secondary structure elements (SSEs), ignoring loops. Dali is based on a sum of pairs score. The score of an alignment involving *n* segments has *n* diagonal terms and *n(n* - 1) off-diagonal terms in the summation. The off-diagonal dependencies pose a difficult combinatorial problem. The branch-and-bound algorithm overcomes the difficulty by initially pooling all possible segment-to-segment pairs as potential constituents of the optimal alignment. An upper bound of the total alignment score is given by the sum of the maxima of each of the $n^2$ terms independently of the others. More formally, the problem of optimizing the alignment score (Eq. 1) over all possible alignments A $\rightarrow$ B can be rewritten using an indicator function $1_{A \rightarrow B}$ as (Eq. 6). Here, the indicator function picks one-to-one correspondences defined by a given alignment A $\rightarrow$ B, while all other terms are zero. The maximum is searched over all possible alignments of m residues in structure A and n residues in structure B, which is a hard combinatorial problem. A partition is a subset of the search space, which can contain many-to-many correspondences between residues in structure A and residues in structure B. An upper bound on the best one-to-one alignment score that is contained within a partition is given by (Eq. 7). The search space is recursively partitioned to derive tighter upper bounds for the subspaces. A binary partition moves one particular query-target segment pairing to one subspace and excludes it from the other. The algorithm terminates when the partition that has the highest upper bound corresponds to unique pairings of all segments.

4. All alignments generated by alignment methods 1-3 above use different objective functions that approximate the Dali score or exclude loops from the alignment. All alignments generated by methods 1-3 are refined using Monte Carlo optimization model, taking into account possible moves into non-optimal territory. The probability of accepting a move is p=exp($\beta$*($S'$-$S$)), where $S'$ is the new score and S is the old score and $\beta$ is a parameter, according to Metropolis, N., Rosenbluth, A. W., Rosenbluth, M. N., Teller, A. H. & Teller, E. (1953) Equation of state calculations by fast computing machines. J. Chem. Phys. 21, 1087-1092.

$$\text{Eq. 1: } S(\mathrm{A},\mathrm{B}) = \sum_{i \in core}\sum_{i \in core}\varphi(i,j)$$

Where S, similarity score, where *i* and *j* label residues, core is the common substructure, and $\varphi$ is a similarity measure based on some pairwise relationship, here on the similarity of intramolecular C$\alpha$-C$\alpha$ distances. Unmatched residues do not contribute to the overall score. For a given functional form of $\varphi$ *(i,j)*, the largest value of S corresponds to the optimal set of residue equivalences. The similarity measure needs to balance two contradictory requirements: maximizing the number of equivalenced residues and minimizing structural deviations. The use of relative rather than absolute deviations of equivalent distances is tolerant to the cumulative effect of gradual geometrical distortions. In Dali, the residue-pair score $\varphi$ has the form:

$$\text{Eq. 2: } \varphi(i,j) = \big(\theta - \mathrm{diff}\ (i,j)\big) * \mathrm{env}(d_{ij}^{*})$$

Where the first term of the multiplication is the relative distance difference compared to a similarity threshold $\theta$ and the second term is an envelope function which down-weights pairs in the long-distance range. In Dali, the similarity threshold is set to $\theta$ = 0.2.

The envelope is a Gaussian function $\mathrm{env}(x) = e^{-\left(\frac{x}{R_0}\right)^2}$ where $R_0$ = 20 A°, calibrated on the size of a typical domain. The relative distance difference diff $(i,j) = \frac{[d_{ij}^{A} + d_{ij}^{B}]}{d_{ij}^{*}}$, where $d_{ij}^{A}$ and $d_{ij}^{B}$ are intramolecular C$\alpha$-C$\alpha$ distances in structure A and B, respectively, and their average is $d_{ij}^{*} = \frac{d_{ij}^{A} + d_{ij}^{B}}{2}$.

Inserting the values of the constants, the resulting raw Dali score describing the structural similarity is given by:

$$\text{Eq. 3: } S(A,B) - \sum_{i \subset core}\sum_{i \subset core}\left(0.2 - \frac{2\left[d_{ij}^{A}\ \ d_{ij}^{B}\right]d}{d_{ij}^{A} \mid d_{ij}^{B}}\right) e^{\left(\frac{d_{ij}^{A} \mid d_{ij}^{B}}{40A}\right)^{2}}$$

The residue-pair scores can get both positive and negative values; therefore, the maximum of Eq. 3 corresponds to a local alignment. Hydrogen bonded backbone segments in helices and sheets have a distance of around 5 A. Here, absolute distance deviations up to 1 A generate positive scores, while larger deviations incur a steeply increasing penalty. At 10 A distance, as found between helices or sheets in tertiary contact, a deviation of up to 2 A still contributes positively, and larger deviations incur a mild penalty. The diameter of a typical domain is around 20 A. Beyond this distance, the score function is relatively insensitive to distance deviations. For example, two conformations of a two-domain structure, which are not superimposable as rigid bodies because of hinge rotation, can be structurally aligned by Dali since the similarity of local structure compensates for the down-weighted deviations in interdomain distances. For random pairwise comparison, the expected Dali score (Eq. 3) increases with the number of residues in the compared proteins. In order to describe the statistical significance of a pairwise comparison score S(A,B), we use the Z-score defined as:

$$\text{Eq. 4: } Z(A,B) = \frac{S(A,B) - m(L)}{\sigma(L)}$$

The relation between the mean score *m,* standard deviation $\sigma$, and the average length $L = \sqrt{L_A L_B}$ of two proteins was derived empirically from a large set of random pairs of structures. Fitting a polynomial gave the approximation:

$$\text{Eq 5: } \begin{array}{c} m(L) \approx 7.95 + 0.71L - 2.59 \cdot 10^{-4}L^2 - 1.92 \cdot 10^{-6}L^3, if\ L \le 400, \\ m(L) - m(100) + L - 400, if\ L > 400 \end{array}$$

$$\text{Eq 6: } S^*(A,B) = \max_{A\to B} \sum_{x=1}^{m} \sum_{y=1}^{m} \sum_{x'=1}^{n} \times \sum_{y'=1}^{n} \varphi(x \to x', y \to y) * \mathbf{1}_{A\to B}(x \to x') * \mathbf{1}_{A\to B}(y \to y')$$

Where $1_x(a) = \begin{cases} 1, \text{if } a \text{ is a member of set } X \\ 0, \text{if } a \text{ is not a member of set } X \end{cases}$

Here, the indicator function picks one-to-one correspondences defined by a given alignment A → B, while all other terms are zero. The maximum is searched over all possible alignments of *m* residues in structure A and *n* residues in structure B, which is a hard combinatorial problem. A partition is a subset of the search space, which can contain manytomany correspondences between residues in structure A and residues in structure B. An upper bound on the best one-to-one alignment score that is contained within a partition is given by

$$\text{Eq. 7: } S^*(\text{partition}) \le \sum_{x=1}^{m} \sum_{y=1}^{m} \max_{\substack{x'=1\ldots n \\ y'=1\ldots n}} (\varphi(x \to x', y \to y'') * \mathbf{1}_{\text{partition}}(x \to x') * \mathbf{1}_{\text{partition}}(y \to y')).$$

The output of DALI labelled "Z-score" measures the extent of similarity. Significant similarities between two proteins have a Z-score above 4, with increasing Z-scores reflecting a higher degree of structural similarity.

Following this approach, 3D structure alignment of WP_179002276.1 MULTISPECIES: hypothetical protein [*Flavobacterium collinsii*], modelled using Alphafold, with PDB entry 6BQS would yield a Z-score of 22.

Treatment composition

The treatment composition comprises (a) from 0.00005 to 5%, preferably from 0.0005 to 2% by weight of the composition, based on active protein, of a porphyrin binding protein. The treatment composition also comprises at least one adjunct component preferably selected from a surfactant, a builder, a bleach component, a polymer, a dispersing agent, and an enzyme. More preferably the composition comprises an enzyme. More preferably, the composition comprises an enzyme and a surfactant. In the case of a dishwashing composition, the composition comprises enzymes

selected from amylases, proteases, and mixtures thereof and preferably a non-ionic surfactant. In the case of a laundry composition, the composition comprises additional enzymes selected from amylases, proteases, lipases, and mixtures thereof and preferably an anionic surfactant.

Benefits that may be associated to the composition of the invention include:

a) reducing or preventing soil redeposition;
b) removal of porphyrin containing soil, especially heme containing soil, such as blood containing soil and/or chlorophyll containing soil, such as grass;
c) facilitating removal of starch-containing soil in the presence of one or more amylases and/or for enhancing amylase related cleaning performance;
d) facilitating removal of protein-containing soil in the presence of one or more proteases and/or for enhancing protease related cleaning performance;
e) facilitating removal of carbohydrase-containing soil in the presence of one or more other carbohydrases and/or enhancing carbohydrase related cleaning performance;
f) facilitating removal of biofilm from an item, such as textile, preferably in a cleaning process such as laundry;
g) cleaning, e.g., deep cleaning of an item, wherein the item is a textile or a surface.

The composition of the present invention may be in the form of a composition for use in a main wash step or as pre-treatment or rinse-added cleaning composition for consumer or institutional use.

Porphyrin binding protein

Porphyrin binding proteins, including hemophores and heme binding proteins function as part of different systems. These include in bacterial nutrient acquisition systems, i.e acquiring heme from hemoglobin and albumin, described in Bateman TJ, Shah M, Ho TP, Shin HE, Pan C, Harris G, Fegan JE, Islam EA, Ahn SK, Hooda Y, Gray-Owen SD, Chen W, Moraes TF. (2021) A Slam-dependent hemophore contributes to heme acquisition in the bacterial pathogen Acinetobacter baumannii. Nat Commun. Nov 1;12(1):6270; and in virulence and cell homeostasis systems, such as described in Wilson T, Mouriño S, Wilks A. (2021) The heme-binding protein PhuS transcriptionally regulates the Pseudomonas aeruginosa tandem sRNA prrF1,F2 locus. J Biol Chem. Jan-Jun;296:100275.

Porphyrin binding proteins can be grouped into different types based on the secretion pathways they are exported through in the host (Shoji M, Sato K, Yukitake H, Kondo Y, Narita Y, et al. (2018) Por Secretion System-Dependent Secretion and Glycosylation of Porphyromonas gingivalis Hemin-Binding Protein 35. PLOS ONE 13(8): e0203154), conserved domains (Grigg JC, Mao CX, Murphy ME. (2011) Iron-coordinating tyrosine is a key determinant of NEAT domain heme transfer. J Mol Biol. Oct 28;413(3):684-98), or their porphyrin binding / heme co-ordination mechanism (Hofbauer S, Pfanzagl V, Michlits H, Schmidt D, Obinger C, Furtmüller PG. Understanding molecular enzymology of porphyrin-binding $\alpha + \beta$ barrel proteins - One fold, multiple functions. Biochim Biophys Acta Proteins Proteom. 2021 Jan; 1869(1): 140536).

Examples of porphyrin binding proteins include HusA-type hemophore like proteins (Gao JL, Nguyen KA, Hunter N. (2010), Characterization of a hemophore-like protein from Porphyromonas gingivalis. J Biol Chem. 285(51):40028-38), HasA-type, HxuA-type, IsdH/NEAT-Type hemophores (reviewed in Wandersman, C. and Delepelaire, P. (2012), Haemophore functions revisited. Molecular Microbiology, 85: 618-631.); HmuY type (Wójtowicz H, Bielecki M, Wojaczyński J, Olczak M, Smalley JW, Olczak T. (2013), The *Porphyromonas gingivalis* HmuY haemophore binds gallium(iii), zinc(ii), cobalt(iii), manganese(iii), nickel(ii), and copper(ii) protoporphyrin IX but in a manner different to iron(iii) protoporphyrin IX. Metallomics.5(4):343-51); Pin-type (Bielecki M, Antonyuk S, Strange RW, Siemińska K, Smalley JW, Mackiewicz P, Śmiga M, Cowan M, Capper MJ, Ślezak P, Olczak M, Olczak T. (2020) *Prevotella intermedia* produces two proteins homologous to *Porphyromonas gingivalis* HmuY but with different heme coordination mode. Biochem J. 477(2):381-405), HphA type (Bateman TJ, Shah M, Ho TP, Shin HE, Pan C, Harris G, Fegan JE, Islam EA, Ahn SK, Hooda Y, Gray-Owen SD, Chen W, Moraes TF. (2021), A Slam-dependent hemophore contributes to heme acquisition in the bacterial pathogen Acinetobacter baumannii. Nat Commun. 12(1):6270), GADPH proteins (Ślęzak P, Śmiga M, Smalley JW, Siemińska K, Olczak T. (2020), *Porphyromonas gingivalis* HmuY and *Streptococcus gordonii* GAPDH-Novel Heme Acquisition Strategy in the Oral Microbiome. Int J Mol Sci. 21(11):4150), Hemoglobin-binding hemophores (Hbp) (Malmirchegini GR, Sjodt M, Shnitkind S, Sawaya MR, Rosinski J, Newton SM, Klebba PE, Clubb RT. (2014), Novel mechanism of hemin capture by Hbp2, the hemoglobin-binding hemophore from Listeria monocytogenes. J Biol Chem. 289(50):34886-99), Hemophillins, HfpY-type (Zhu Y, Lechardeur D, Bernardet JF, Kerouault B, Guérin C, Rigaudeau D, Nicolas P, Duchaud E, Rochat T. (2022), Two functionally distinct heme/iron transport systems are virulence determinants of the fish pathogen Flavobacterium psychrophilum. Virulence. 13(1):1221-1241) and Rv0203 heme-transport proteins (Owens CP, Chim N, Graves AB, Harmston CA, Iniguez A, Contreras H, Liptak MD, Goulding CW. (2013) The Mycobacterium tuberculosis secreted protein Rv0203 transfers heme to membrane proteins MmpL3 and MmpL11. J Biol Che. 288(30):21714-28).

Preferably the porphyrin binding protein is present in the aqueous wash liquor in an amount of from 0.01ppm to 1000 ppm of theprotein, or from 0.05 or from 0.1ppm to 750 or 500ppm.

Preferably the porphyrin binding protein is present in the composition in an amount from 0.00005 to 5 wt.% based on active protein in the composition, or 0.001 to 1 wt.%, or from 0.005 to 0.5 wt.% or from 0.01 to 0.25 wt.% based on weight of the composition.

The composition comprises an optional additional adjunct. Typically, the additional adjunct will be present in the composition in an amount from 1 to 98.9 wt.%, more typically from 5 to 90 wt.% adjunct. Suitable additional adjuncts comprise: colorants, chelating agents, dye transfer agents, deposition aids, dispersants, enzyme stabilizers, catalytic materials, optical brighteners, photoactivators, fluorescers, fabric hueing agents (shading dyes), fabric conditioners, preformed peracids, polymeric dispersing agents, clay soil removal/anti-redeposition agents, filler salts, hydrotropes, brighteners, suds suppressors, structure elasticizing agents, fabric softeners, preservatives, anti-oxidants, anti-shrinkage agents, germicides, fungicides, anti-tarnish, anti-corrosion agents, alkalinity sources, solubilizing agents, carriers, processing aids, pigments, dyes, perfumes and pH control agents, encapsulates, polymers and mixtures thereof. For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; fabric-softeners such as clay, silicone and/or quaternary ammonium compounds; flocculants such as polyethylene oxide; dye transfer inhibitors such as polyvinylpyrrolidone, poly 4-vinylpyridine N-oxide and/or co-polymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes such as perfume microcapsules, starch encapsulated accords, perfume spray-on; soap rings; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; diethylene glycol, ethanol, and any mixture thereof; hydrotropes such as sodium cumene sulphonate, sodium xylene sulphonate, sodium toluene sulphonate, and any mixtures; organic acids and salts thereof, such as citric acid/citrate salts; and any combination thereof. The composition may be such that the cleaning adjunct comprises one or more selected from the group consisting of (i) perfume microcapsule; (ii) fabric hueing agent; (iii) protease; (iv) amphiphilic cleaning polymer; (v) amylase, or (vi) mixtures thereof.

Anionic Surfactant

The composition of the invention can provide good soil breakdown, however the removal of the products of the breakdown of the substrates and soils containing them is improved by the presence of anionic surfactant. Therefore, the laundry composition preferably comprises from 1 to 60 wt% an anionic surfactant. Preferably the weight ratio of anionic surfactant to active enzyme protein of the invention is at least 500:1, preferably at least 1000:1 or at least 1500:1 or at least 2000:1, preferably being no greater than 500000:1, preferably no greater than 400000:1, or no greater than 200000:1 or up to 150000:1 or 100000:1, or 50000:1 or 10000:1.

Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, C8-C16 ester sulfates and C10-C14 ester sulfates, such as mono dodecyl ester sulfates. In a preferred composition the anionic surfactant comprises alkyl benzene sulphonate and optionally in addition, optionally ethoxylated alkyl sulfate, preferably having a degree of ethoxylation from 0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof are also suitable anionic surfactants.

The surfactant preferably comprises a surfactant system comprising an anionic surfactant and in addition, one or

more additional surfactants, which may be non-ionic including semi-polar and/or cationic and/or zwitterionic and/or ampholytic and/or amphoteric and/or semi-polar nonionic and/or mixtures thereof.

The invention also provides a cleaning composition comprising: from 0.00005 to 5 wt% (active protein) of the porphyrin binding protein of the invention and a surfactant wherein the surfactant comprises an anionic and a nonionic surfactant, preferably having a weight ratio of anionic to nonionic of from 30:1 to 1:2, preferably from 20:1 to 2:3 or to 1:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac ™, Lutensol™ and Pluronic™ from BASF, Dehypon™ series from Cognis and Genapol™ series from Clariant.

The composition preferably comprises from 0.5wt% to about 40wt% of a non-ionic surfactant, preferably 1 to 30 wt.% of the composition non-ionic surfactant.

The composition preferably comprises one or more enzymes selected from the group consisting of aminopeptidase, amylase, carbohydrase, carboxypeptidase, catalase, cellulase, chitinase, cutinase, cyclodextrin glycosyltransferase, esterase, alpha-galactosidase, beta-galactosidase, glucoamylase, alpha-glucosidase, beta-glucosidase, haloperoxidase, hexosaminidase, invertase, laccase, lipase, mannanase, mannosidase, nuclease, oxidase, pectinolytic enzyme, peptidoglutaminase, peroxidase, phytase, polyphenoloxidase, proteolytic enzyme, transglutaminase, xylanase, xanthan lyase, xanthanase, endo-β-1,3-glucanase and mixtures thereof. Preferably the cleaning composition comprises an enzyme selected from nuclease, such as DNase or RNase, mannanase, xanthan lyase, xanthanase, amylase and mixtures thereof.

Preferably the composition comprises enzymes selected from xanthan lyase, xanthanase, mannanase and mixtures thereof. Mannanase is particularly preferred.

The enzyme(s) may be produced, for example, by a microorganism belonging to the genus *Aspergillus, e.g., Aspergillus aculeatus, Aspergillus awamori, Aspergillus foetidus, Aspergillus fumigatus, Aspergillus japonicus, Aspergillus nidulans, Aspergillus niger,* or *Aspergillus oryzae; Fusarium, e.g., Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum; Humicola, e.g., Humicola insolens* or *Humicola lanuginosa;* or *Trichoderma, e.g., Trichoderma harzianum, Trichoderma koningii, Trichoderma longibrachiatum, Trichoderma reesei,* or *Trichoderma viride.*

Preferably the composition comprises a protease or mixture of more than one protease and one or more amylolytic enzymes, e.g., an alpha-amylase, glucoamylase, maltogenic amylase. In the case of laundry compositions, the composition can optionally comprise lipase or mixture of more than one lipase, a peroxidase or mixture of more than one peroxidase, and/or a cellulase or mixture thereof.

In general, the properties of the chosen enzyme(s) should be compatible with the selected composition, (i.e., pH-optimum, compatibility with other enzymatic and non-enzymatic ingredients, etc.), and the enzyme(s) should be present in effective amounts. Preferably, the composition of the invention comprises at least 0.01 mg, preferably from about 0.05 to about 10, more preferably from about 0.1 to about 6, especially from about 0.2 to about 5 mg of active further enzyme/ g of composition.

Proteases: The composition of the invention preferably comprises a protease. A mixture of two or more proteases can contribute to an enhanced cleaning across a broader temperature, cycle duration, and/or substrate range. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:

Subtilisins (EC 3.4.21.62), especially those derived from *Bacillus,* such as *Bacillus sp., B. lentus, B. alkalophilus, B. subtilis, B. amyloliquefaciens, B. pumilus, B. gibsonii,* and *B. akibai* described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569 and WO2016174234. Specifically, mutations S9R, A15T, V66A, A188P, V199I, Q239R, N255D (savinase numbering system).

Trypsin-type or chymotrypsin-type proteases, such as trypsin (e.g., of porcine or bovine origin), including the

Fusarium protease described in WO 89/06270 and the chymotrypsin proteases derived from *Cellumonas* described in WO 05/052161 and WO 05/052146.

Metalloproteases, especially those derived from *Bacillus amyloliquefaciens* decribed in WO07/044993A2; from *Bacillus, Brevibacillus, Thermoactinomyces, Geobacillus, Paenibacillus, Lysinibacillus* or *Streptomyces spp.* described in WO2014194032, WO2014194054 and WO2014194117; from *Kribella alluminosa* described in WO2015193488; and from *Streptomyces* and *Lysobacter* described in WO2016075078.

Protease having at least 90% identity to the subtilase from *Bacillus sp.* TY145, NCIMB 40339, described in WO92/17577 (Novozymes A/S), including the variants of this *Bacillus sp* TY145 subtilase described in WO2015024739, and WO2016066757.

Especially preferred proteases for the composition of the invention are polypeptides having at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Most preferably the protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).

(i) G118V + S128L + P129Q + S130A

(ii) S101M + G118V + S128L + P129Q + S130A

(iii) N76D + N87R + G118R + S128L + P129Q + S130A + S188D + N248R

(iv) N76D + N87R + G118R + S128L + P129Q + S130A + S188D + V244R

(v) N76D + N87R + G118R + S128L + P129Q + S130A

(vi) V68A + N87S + S101G + V104N

(vii) S99AD

(viii) S9R+A15T+V68A+N218D+Q245R

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase®, Coronase®, Blaze®, Blaze Ultra® and Esperase® by Novozymes A/S (Denmark); those sold under the tradename Maxatase®, Maxacal®, Maxapem®, Properase®, Purafect®, Purafect Prime®, Purafect Ox®, FN3®, FN4®, Excellase®, Ultimase® and Purafect OXP® by Dupont; those sold under the tradename Opticlean® and Optimase® by Solvay Enzymes; and those available from Henkel/Kemira, namely BLAP (sequence shown in Figure29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and KAP (Bacillus alkalophilus subtilisin with mutations A230V + S256G + S259N) from Kao.

Especially preferred for use herein are commercial proteases selected from the group consisting of Properase®, Blaze®, Ultimase®, Everlase®, Savinase®, Excellase®, Blaze Ultra®, BLAP and BLAP variants.

Preferred levels of protease in the composition of the invention include from about 0.05 to about 10, more preferably from about 0.5 to about 7 and especially from about 1 to about 6 mg of active protease/g of composition.

Lipases: The composition preferably comprises a lipase. The presence of oils and/or grease can further increase the resiliency of stains comprising mannans and other polysaccharides. As such, the presence of lipase in the enzyme package can further improve the removal of such stains. Suitable lipases include those of bacterial or fungal or synthetic origin. Chemically modified or protein engineered mutants are included. Examples of useful lipases include lipases from *Humicola* (synonym *Thermomyces),* e.g., from *H. lanuginosa (T. lanuginosus*) or from *H. insolens,* a *Pseudomonas lipase,* e.g., from *P. alcaligenes* or *P. pseudoalcaligenes, P. cepacia P. stutzeri, P. fluorescens, Pseudomonas* sp. strain SD 705, *P. wisconsinensis* , a *Bacillus* lipase, e.g., from *B. subtilis* (Dartois et al. (1993), Biochemica et Biophysica Acta, 1131, 253-360), *B. stearothermophilus* or *B. pumilus* .

The lipase may be a "first cycle lipase" such as those described in U.S. Patent 6,939,702 B1 and US PA 2009/0217464. In one aspect, the lipase is a first-wash lipase, preferably a variant of the wild-type lipase from *Thermomyces lanuginosus* comprising T231R and N233R mutations. The wild-type sequence is the 269 amino acids (amino acids 23 - 291) of the Swissprot accession number Swiss-Prot O59952 (derived from *Thermomyces lanuginosus (Humicola lanuginosa))*. Preferred lipases include those sold under the tradenames Lipex®, Lipolex® and Lipoclean®.

Other suitable lipases include: Liprl 139, e.g., as described in WO2013/171241; TfuLip2, e.g., as described in WO2011/084412 and WO2013/033318; Pseudomonas stutzeri lipase, e.g., as described in WO2018228880; *Microbulbifer thermotolerans* lipase, e.g., as described in WO2018228881; *Sulfobacillus acidocaldarius* lipase, e.g., as described in EP3299457; LIP062 lipase e.g., as described in WO2018209026; PinLip lipase e.g., as described in WO2017036901 and Absidia sp. lipase e.g., as described in WO2017005798.

Suitable lipases are commercially available from Novozymes, for example as Lipex Evity 100L, Lipex Evity 200L (both liquid raw materials) and Lipex Evity 105T (a granulate). These lipases have different structures to the products Lipex 100L, Lipex 100T and Lipex Evity 100T which are outside the scope of the invention.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.* disclosed in US 4,435,307, US 5,648,263 , US 5,691,178, US 5,776,757 and US 5,691,178 .

In one aspect, preferred enzymes include microbial-derived endoglucanases exhibiting endo-beta-1,4-glucanase activity (E.C. 3.2.1.4), preferably selected from the group comprising:

(a) a bacterial polypeptide endogenous to a member of the genus Bacillus which has a sequence of at least 90%, 94%, 97% and even 99% identity to the amino acid sequence SEQ ID NO:2 in US 7,141,403B2, preferred substitutions compriseone or more positions corresponding to positions 292, 274, 266, 26 5, 255, 246, 237, 224 and 221 of the mature polypeptide of SEQ ID NO: 2, a nd the variant has cellulase activity.;
(b)a glycosyl hydrolase having enzymatic activity towards both xyloglucan and amorphous cellulose substrates, wherein the glycosyl hydrolase is selected from GH families 5, 7, 12, 16, 44 or 74;
(c) a glycosyl hydrolase having a sequence of at least 90%, 94%, 97% and even 99% identity to the amino acid sequence SEQ ID NO:3 in WO09/148983;
(d) Variants exhibiting at least 70% identity with SEQ ID NO: 5 in WO2017106676. Preferred substitutions comprise one or more positions corresponding to positions 4, 20, 23, 29, 32, 36, 44, 51, 77, 80, 87, 90, 97, 98, 99, 102, 112, 116, 135, 136, 142, 153, 154, 157, 161, 163, 192, 194, 204, 208, 210, 212, 216, 217, 221, 222, 225, 227, and 232;
(e) and mixtures thereof.

Suitable endoglucanases are sold under the tradenames Celluclean® and Whitezyme® (Novozymes A/S, Bagsvaerd, Denmark). Examples include Celluclean® 5000L, Celluclean® Classic 400L, Celluclean® Classic 700T, Celluclean® 4500T, Whitezyme® 1.5T, Whitezyme® 2.0L.

Other commercially available cellulases include Celluzyme®, Carezyme®, Carezyme® Premium (Novozymes A/S), Clazinase®, Puradax HA®, Revitalenz® 1000, Revitalenz® 2000 (Genencor International Inc.), KAC-500(B)® (Kao Corporation), Biotouch® FCL, Biotouch® DCL, Biotouch® DCC, Biotouch® NCD, Biotouch® FCC, Biotouch® FLX1 (AB Enzymes)

Suitable glucanases include endo-β-1,3-glucanases, preferably from E.C. class 3.2.1.39, preferably obtained from *Paenibacillus sp, Zobellia galactanivorans, Thermotoga petrophila* or *Trichoderma sp* micro-organism, preferably *Paenibacillus sp* or *Zobellia galactanivorans,* most preferably *Paenibacillus sp.*

Amylases: Preferably the composition of the invention comprises an amylase. Suitable alpha-amylases include those of bacterial or fungal origin. Chemically or genetically modified mutants (variants) are included. A preferred alkaline alpha-amylase is derived from a strain of Bacillus, such as *Bacillus licheniformis, Bacillus amyloliquefaciens, Bacillus stearothermophilus, Bacillus subtilis,* or other *Bacillus sp.,* such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:

(a) variants described in USP 5,856,164 and WO99/23211, WO 96/23873, WO00/60060, WO06/002643 and WO2017/192657, especially the variants with one or more substitutions in the following positions versus the AA560 enzyme listed as SEQ ID No. 12 in WO 06/002643:
26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.
(b) variants exhibiting at least 85%, preferably 90% identity with SEQ ID No. 4 in WO06/002643, the wild-type enzyme

from Bacillus SP722, especially variants with deletions in the 183 and 184 positions and variants described in WO 00/60060, WO2011/100410 and WO2013/003659, particularly those with one or more substitutions at the following positions versus SEQ ID No. 4 in WO06/002643 which are incorporated herein by reference: 51, 52, 54, 109, 304, 140, 189, 134, 195, 206, 243, 260, 262, 284, 347, 439, 469, 476 and 477.

(c) variants exhibiting at least 90% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations. Additional relevant mutations/deletions based on SP707 backbone include W48, A51, V103, V104, A113, R118, N125, V131, T132, E134, T136, E138, R142, S154, V165, R182, G182, H183, E190, D192, T193, I206, M208, D209, E212, V213, V214, N214, L217, R218, N219, V222, T225, T227, G229, I235, K242, Y243, S244, F245, T246, I250, S255, A256, H286, V291, T316, V317, V318, N417, T418, A419, H420, P421, I428, M429, F440, R443, N444, K445, Q448, S451, A465, N470, S472.

(d) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.

(e) variants described in WO10/115021, especially those exhibiting at least 75%, or at least 85% or at least 90% or at least 95% with SEQ ID NO:2 in WO10/115021, the alpha-amylase derived from *Bacillus sp.* TS-23.

(f) variants exhibiting at least 89% identity with SEQ ID NO:1 in WO2016091688, especially those comprising deletions at positions H183+G184 and additionally one or more mutations at positions 405, 421, 422 and/or 428.

(g) variants described in WO2014099523, especially those exhibiting at least 60% amino acid sequence identity with the "PcuAmyl $\alpha$-amylase" from *Paenibacillus curdlanolyticus* YK9 (SEQ ID NO:3 in WO2014099523).

(h) variants described in WO2014099523, especially those exhibiting at least 60% amino acid sequence identity with the "CspAmy2 amylase" from *Cytophaga sp.* (SEQ ID NO:1 & 6 in WO2014164777. Especially those comprising one of more of the following deletions and/or mutations based on SEQ ID NO:1 in WO2014164777: R178*, G179*, T38N, N88H, N126Y, T129I, N134M, F153W, L171R, T180D, E187P, I203Y, G476K, G477E, Y303D.

(i) variants exhibiting at least 85% identity with AmyE from *Bacillus subtilis* (SEQ ID NO:1 in WO2009149271).

(j) variants exhibiting at least 90% identity with the wild-type amylase from *Bacillus sp.* KSM-K38 with accession number AB051102.

(k) variants described in WO2016180748, especially those exhibiting at least 80% identity with the mature amino acid sequence of AAI10 from *Bacillus sp* in SEQ ID NO: 7 in WO2016180748; those exhibiting at least 80% identity with the mature amino acid sequence of *Alicyclobacillus sp.* amylase in SEQ ID NO: 8 in WO2016180748, and those exhibiting at least 80% identity with the mature amino acid sequence of SEQ ID NO: 13 in WO2016180748, especially those comprising one or more of the following mutations H*, N54S, V56T, K72R, G109A, F113Q, R116Q, W167F, Q172G, A174S, G184T, N195F, V206L, K391A, P473R, G476K.

(l) variants described in WO2018060216, especially those exhibiting at least 70% identity with the mature amino acid sequence of SEQ ID NO: 4 in WO2018060216, the fusion molecule of *Bacillus amyloliquefaciens* and *Bacillus licheniformis.* Especially those comprising one or more substitutions at positions H1, N54, V56, K72, G109, F113, R116, T134, W140, W159, W167, Q169, Q172, L173, A174, R181, G182, D183, G184, W189, E194, N195, V206, G255, N260, F262, A265, W284, F289, S304, G305, W347, K391, Q395, W439, W469, R444, F473, G476, and G477.

Preferred amylases are engineered enzymes, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in SEQ ID NO: 11 is substituted. Preferably, the methionine at this position is substituted with threonine or leucine, preferably leucine.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL®, ATLANTIC®, ACHIEVE ALPHA®, AMPLIFY® PRIME, INTENSA® and BAN® (Novozymes A/S, Bagsvaerd, Denmark), KEMZYM® AT 9000 Biozym Biotech Trading GmbH Wehlistrasse 27b A-1200 Wien Austria, RAPIDASE®, PURASTAR®, ENZYSIZE®, OPTISIZE HT PLUS®, POWERASE®, PREFERENZ S® series (including PREFERENZ S1000® and PREFERENZ S2000® and PURASTAR OXAM® (DuPont., Palo Alto, California) and KAM® (Kao, 14-10 Nihonbashi Kayabacho, 1-chome, Chuo-ku Tokyo 103-8210, Japan).

Preferably, the composition comprises at least 0.01 mg, preferably from about 0.05 to about 10, more preferably from about 0.1 to about 6, especially from about 0.2 to about 5 mg of active amylase/ g of composition.

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from C. *cinereus,* and variants thereof as those described in WO 93/24618, WO 95/10602, and WO 98/15257.

Commercially available peroxidases include GUARDZYME® (Novozymes A/S).

Pectate lyase: Suitable pectate lyases include those sold under the tradenames Pectawash®, Pectaway®, X-Pect®, (all Novozymes A/S, Bagsvaerd, Denmark) Preferenz® F1000 (DuPont Industrial Biosciences).

Mannanases. The composition preferably comprises one of more mannanase enzymes. As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78 and belong in Glycosyl Hydrolase families 5, 26 and 113. Many suitable mannanases belong to Glycosyl Hydrolase family 5. Commercially available mannanases include all those sold under the tradenames Mannaway® (Novozymes A/S) such as Mannaway® 200L and Mannaway Evity 4.0T Other commercially available mannanases include Effectenz® M1000, Mannastar® 375, Preferenz M100 and Purabrite® (all DuPont Industrial Biosciences) and Biotouch M7 (AB Enzymes). Other suitable mannanases belong to Glycosyl Hydrolase family 26 including those described in WO2018191135, WO2015040159, WO2017021515, WO2017021516, WO2017021517 and WO2019081515. Suitable mixtures of mannanases include the combinations of Glycosyl Hydrolase family 5 and Glycosyl Hydrolase family 26 mannanases described in WO2019081515.

Nucleases: Preferably the composition comprises a nuclease enzyme such as a RNase or DNase or mixtures thereof. The nuclease enzyme is an enzyme capable of cleaving the phosphodiester bonds between the nucleotide subunits of nucleic acids. The nuclease enzyme herein is preferably a deoxyribonuclease or ribonuclease enzyme or a functional fragment thereof. By functional fragment or part is meant the portion of the nuclease enzyme that catalyzes the cleavage of phosphodiester linkages in the DNA backbone and so is a region of said nuclease protein that retains catalytic activity. Thus, it includes truncated, but functional versions, of the enzyme and/or variants and/or derivatives and/or homologues whose functionality is maintained.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1 and mixtures thereof.

DNase: Suitable DNases include wild-types and variants of DNases defined by SEQ ID NOS: 1, 2, 3, 4, 5, 6, 7, 8 and 9 in WO2017162836 (Novozymes), and SEQ ID NOS: 1, 2, 3, 4, 5, 6, 7, 8, 9, 10, 11, 12, 13, 14, 15, 16, 17, 18, 19, 20, 21, 22, 23, 24, 25, 28, 29, 30, 31, 32, 33, 34, 35, 36, 37, 38 39, 40, 41, 42, 43, 44, 45, 46, 47, 48, 49 50, 51, 52, 53, and 54 in WO2018108865, and variants of the *Bacillus cibi* DNase including those described in WO2018011277 (Novozymes), incorporated herein by reference. Preferred DNases are as claimed in EP3476935A.

RNase: suitable RNases include wild-types and variants of DNases defined by SEQ ID NOS: 3, 6, 9, 12, 15, 57, 58, 59, 60, 61, 62, 63, 64, 65, 66, 67, 72 and 73 in WO2018178061 (Novozymes), and SEQ ID NOS: 86, 87, 88, 89, 90, 91, 92, 93, 94, 95, 96, 97, 98, 99, 100, 101, 102, 103 and 104 in WO2020074499 (Novozymes), incorporated herein by reference.

Hexosaminidase: The composition preferably additionally comprises one or more hexosaminidase enzymes. The term hexosaminidase includes "dispersin" and the abbreviation "Dsp", which means a polypeptide having hexosaminidase activity. Suitable enzymes are found in EC 3.2.1.- that catalyze the hydrolysis of β-1,6-glycosidic linkages of N-acetylglucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944.

Variants of the *Terribacillus saccharophilus* hexosaminidase defined by SEQ ID NO: 1 of WO2020207944 are preferred, especially the variants with improved thermostability disclosed in that publication.

Particularly preferred hexosaminidase enzymes are hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 8; and hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 9 disclosed in WO2022/094164; and mixtures thereof.

Alpha-1, 4-polygalactosaminidase: The composition preferably comprises one or more alpha-1, 4-polygalactosaminidase enzymes (EC 3.2.1.109). The term alpha-1, 4-polygalactosaminidase includes "Pel-ase", Which means a polypeptide having activity against alpha-1,4-polygalactosamine residues, that can be further partially N-acetylated. Pel-ase activity may be determined by activity towards poly-galactosamine substrate according to assay described in WO2019228448. Suitable Pel-ases include polypeptides comprising a GH114 domain having hydrolytic activity towards

poly-galactosamine, including those disclosed in WO2018102478, WO2019228448, WO2018185181, WO2020070011, WO2018185267, WO2020070209 or WO2020008043; and mixtures thereof.

The composition preferably comprises one or more enzymes having glycoside hydrolase activity and selected from glycoside hydrolase family GH 39, termed Psl-ases. Wherein the glycoside hydrolase comprises PslGh comprising a GH39 glycosyl hydrolase domain. Suitable Psl-ases include those disclosed in WO 2018102479, WO2018185280, WO2020008043; and mixtures thereof.

The composition preferably comprises one or more alginate lyase enzymes. The term alginate lyase includes polypeptides wherein the alginate lyase enzyme has activity towards poly(beta-D-mannuronate) (polyM activity) and/or activity towards poly(alpha-L-guluronate) (polyG activity) substrates. Alginate lyase activity may be determined according to assay described in WO2022094588. Suitable alginate lyases include those disclosed in WO2022094588 and WO2022094163.

Xanthan gum-degrading enzymes: The composition may comprise one of more xanthan gum-degrading enzymes. Suitable enzymes for degradation of xanthan gum-based soils include xanthan endoglucanase, optionally in conjunction with a xanthan lyase. As used herein, the term "xanthan endoglucanase" denotes an enzyme exhibiting endo-β-1,4-glucanase activity that is capable of catalysing hydrolysis of the 1,4-linked β-D-glucose polymeric backbone of xanthan gum, optionally in conjunction with a suitable xanthan lyase enzyme. Suitable xanthan endoglucanases are described in WO2013167581, WO2015181299, WO2015181292, WO2017046232, WO2017046260, WO201837062, WO201837065, WO2019038059 and WO2019162000. As used herein, the term "xanthan lyase" denotes an enzyme that cleaves the β-D-mannosyl-β-D-1,4-glucuronosyl bond of xanthan gum. Such enzymes belong to E.C. 4.2.2.12. Suitable xanthan lyases are described in WO2015001017, WO2018037061, WO201837064, WO2019038060, WO2019162000 and WO2019038057.

Galactanase: Preferably the composition comprises a galactanase, ie. an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

The 1 enzyme(s) may be included in composition by adding separate enzyme additives containing an additional enzyme, or a combined enzyme additive comprising two or several or all of the additional enzymes. Such an enzyme additive can be in the form of a granulate, a liquid or slurry, preferably additionally comprising an enzyme stabiliser.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Fabric Hueing Agent. The composition may comprise a fabric hueing agent (sometimes referred to as shading, bluing or whitening agents/dyes). Typically the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Preferred are azo dyes, especially mono- or bis- azo dyes, triarylmethane dyes and anthraquinone dyes.

Suitable fabric hueing agents include dyes, dye-clay conjugates, and organic and inorganic pigments. Suitable dyes include small molecule dyes and polymeric dyes. Suitable small molecule dyes include small molecule dyes selected from the group consisting of dyes falling into the Colour Index (C.I.) classifications of Direct, Basic, Reactive or hydrolysed Reactive, Solvent or Disperse dyes. Examples of suitable small molecule dyes include for example small molecule dyes selected from the group consisting of Colour Index (Society of Dyers and Colourists, Bradford, UK) numbers Direct Violet dyes such as 9, 35, 48, 51, 66, and 99, Direct Blue dyes such as 1, 71, 80 and 279, Acid Red dyes such as 17, 73, 52, 88 and 150, Acid Violet dyes such as 15, 17, 24, 43, 49, 50 and 51, Acid Blue dyes such as 15, 17, 25, 29, 40, 45, 75, 80, 83, 90 and 113, Acid Black dyes such as 1, Basic Violet dyes such as 1, 3, 4, 10 and 35, Basic Blue dyes such as 3, 16, 22, 47, 66, 75 and 159, Disperse or Solvent dyes such as those described in EP1794275 or EP1794276, or dyes as disclosed in US 7,208,459 B2,and mixtures thereof.

Preferred are polymeric dyes include polymeric dyes selected from the group consisting of polymers containing covalently bound (sometimes referred to as conjugated) chromogens, (dye-polymer conjugates), for example polymers with chromogens co-polymerized into the backbone of the polymer and mixtures thereof. Polymeric dyes include those

described in WO2011/98355, WO2011/47987, US2012/090102, WO2010/145887, WO2006/055787 and WO2010/142503.

Preferred polymeric dyes comprise alkoxylated, preferably ethoxylated azo, anthraquinone or triarylmethane dyes. Ethoxylated thiophene azo dyes are especially preferred, for example polymeric dyes selected from the group consisting of fabric-substantive colorants sold under the name of Liquitint® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. Suitable polymeric dyes include polymeric dyes selected from the group consisting of Liquitint® Violet CT, carboxymethyl cellulose (CMC) covalently bound to a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenylmethane polymeric colourants, alkoxylated thiophene polymeric colourants, and mixtures thereof.

Preferred hueing dyes include the alkoxylated thiophene azo whitening agents found in US2008/0177090 which may be optionally anionic, such as those selected from Examples 1-42 in Table 5 of WO2011/011799. Other preferred dyes are disclosed in US 8138222.

Suitable pigments include pigments selected from the group consisting of Ultramarine Blue (C.I. Pigment Blue 29), Ultramarine Violet (C.I. Pigment Violet 15) and mixtures thereof. Pigments and/or dyes may also be added to add colour for aesthetic reasons. Preferred are organic blue, violet and/or green pigments.

Builders: The composition may further contain builders, such as builders based on carbonate, bicarbonate or silicates which may be Zeolites, such as Zeolite A, Zeolite MAP (Maximum Aluminium type P). Zeolites, useable in laundry preferably has the formula $Na_{12}(AlO_2)_{12}(SiO_2)_{12}\cdot 27H_2O$ and the particle size is usually between 1-10 $\mu$m for zeolite A and 0.7-2 um for zeolite MAP. Other builders are Sodium metasilicate ($Na_2SiO_3 \cdot nH_2O$ or $Na_2Si_2O_5 \cdot n\ H_2O$) strong alkaline and preferably used in dish wash. In preferred embodiments, the amount of a detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof. Preferred encapsulates comprise a core comprising perfume. Such encapsulates are perfume microcapsules.

Enzyme stabilizer: The composition may comprise an enzyme stabilizer. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof.

Structurant: In one aspect, the composition may comprise a structurant selected from the group consisting of diglycerides and triglycerides, ethylene glycol distearate microcrystalline cellulose, cellulose-based materials, microfiber cellulose, biopolymers, xanthan gum, gellan gum, and mixtures thereof.

Polymers: The composition preferably comprises one or more polymers. Preferred examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers and amphiphilic polymers and mixtures thereof.

Amphiphilic cleaning polymers: Preferably, the amphiphilic cleanimg polymer is a compound having the following general structure: bis($(C_2H_5O)(C_2H_4O)n)(CH_3)$-$N^+$-$C_xH_{2x}$-$N^+$-$(CH_3)$-bis($(C_2H_5O)(C_2H_4O)n$), wherein n = from 20 to 30, and x = from 3 to 8, or sulphated or sulphonated variants thereof.

Amphiphilic alkoxylated grease cleaning polymers suitable for use in the composition of the invention refer to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

The core structure may comprise a polyalkylenimine structure comprising, in condensed form, repeating units of formulae (I), (II), (III) and (IV):

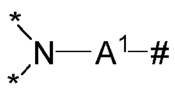
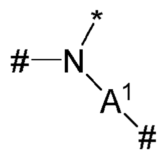
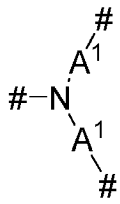
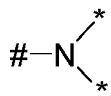

(I) (II) (III) (IV)

wherein # in each case denotes one-half of a bond between a nitrogen atom and the free binding position of a group $A^1$ of two adjacent repeating units of formulae (I), (II), (III) or (IV); * in each case denotes one-half of a bond to one of the alkoxylate groups; and $A^1$ is independently selected from linear or branched $C_2$-$C_6$-alkylene; wherein the polyalkylenimine structure consists of 1 repeating unit of formula (I), x repeating units of formula (II), y repeating units of formula (III) and y+1 repeating units of formula (IV), wherein x and y in each case have a value in the range of from 0 to about 150; where the average weight average molecular weight, Mw, of the polyalkylenimine core structure is a value in the range of from about 60 to about 10,000 g/mol.

The core structure may alternatively comprise a polyalkanolamine structure of the condensation products of at least one compound selected from N-(hydroxyalkyl)amines of formulae (I.a) and/or (I.b),

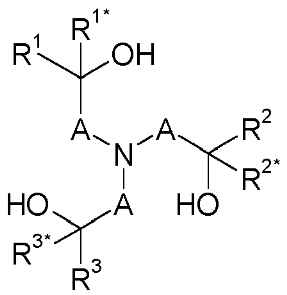
(I.a)

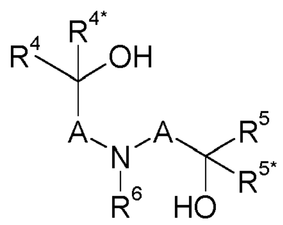
(I.b)

wherein A are independently selected from $C_1$-$C_6$-alkylene; $R^1$, $R^{1*}$, $R^2$, $R^{2*}$, $R^3$, $R^{3*}$, $R^4$, $R^{4*}$, $R^5$ and $R^{5*}$ are independently selected from hydrogen, alkyl, cycloalkyl or aryl, wherein the last three mentioned radicals may be optionally substituted; and $R^6$ is selected from hydrogen, alkyl, cycloalkyl or aryl, wherein the last three mentioned radicals may be optionally substituted.

The plurality of alkylenoxy groups attached to the core structure are independently selected from alkylenoxy units of the formula (V)

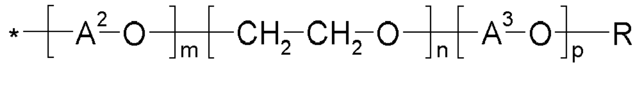

(V)

wherein * in each case denotes one-half of a bond to the nitrogen atom of the repeating unit of formula (I), (II) or (IV); $A^2$ is in each case independently selected from 1,2-propylene, 1,2-butylene and 1,2-isobutylene; $A^3$ is 1,2-propylene; R is in each case independently selected from hydrogen and $C_1$-$C_4$-alkyl; m has an average value in the range of from 0 to about 2; n has an average value in the range of from about 20 to about 50; and p has an average value in the range of from about 10 to about 50.

Carboxylate polymer: The composition preferably also includes one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers having a structure as defined by one of the following structures (I), (II) or (III):

(I) $-[(OCHR^1-CHR^2)_a-O-OC-Ar-CO-]_d$
(II) $-[(OCHR^3-CHR^4)_b-O-OC-sAr-CO-]_e$
(III) $-[(OCHR^5-CHR^6)_c-OR^7]_f$

wherein:

a, b and c are from 1 to 200;
d, e and f are from 1 to 50;
Ar is a 1,4-substituted phenylene;
sAr is 1,3-substituted phenylene substituted in position 5 with $SO_3Me$;
Me is Li, K, Mg/2, Ca/2, Al/3, ammonium, mono-, di-, tri-, or tetraalkylammonium wherein the alkyl groups are $C_1$-$C_{18}$ alkyl or $C_2$-$C_{10}$ hydroxyalkyl, or mixtures thereof;
$R^1$, $R^2$, $R^3$, $R^4$, $R^5$ and $R^6$ are independently selected from H or $C_1$-$C_{18}$ n- or iso-alkyl; and
$R^7$ is a linear or branched $C_1$-$C_{18}$ alkyl, or a linear or branched $C_2$-$C_{30}$ alkenyl, or a cycloalkyl group with 5 to 9 carbon atoms, or a $C_8$-$C_{30}$ aryl group, or a $C_6$-$C_{30}$ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleaching system: The composition may contain a bleaching system, for example comprising a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; and salts derivatives thereof and mixtures thereof. Preferred chelants are selected from the group consisting of methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

The composition may also contain other conventional detergent ingredients such as e.g., fabric conditioners including clays, foam boosters, suds suppressors, anti-corrosion agents, soil-suspending agents, anti-soil re-deposition agents, dyes, bactericides, optical brighteners, hydrotropes, tarnish inhibitors, organic solvents such as ethanol or perfumes.

METHODS

The present invention also provides a method for treating a substrate, preferably a method for cleaning a

substrate. The substrate can be a soft surface, such as a fabric or a hard surface, such as cookware and/or tableware. The method comprises a contacting step, contacting a surface preferably with an aqueous wash liquor comprising the protein of the invention, preferably in an amount from 0.01ppm to 10ppm, preferably from 0.1ppm to 1ppm; and preferably additionally a surfactant, preferably in an amount from 0.05 to 50g/l, more preferably from 0.2g/l to 5g/l or 0.5g/l to 3g/l.

The aqueous wash liquor may be formed by adding a composition as described above to water, for example in a washing machine, dishwasher or hand washing process. The surface may be optionally subsequently washed, and/or rinsed and/or dried.

The porphyrin binding protein may be present in the wash liquor in an amount corresponding to 0.001-100 mg of active protein per litre of wash liquor, preferably 0.005-5 mg of active protein per litre of wash liquor, more preferably 0.01-1 mg of protein per litre of wash liquor and in particular 0.1-1 mg of protein per litre of wash.

In the contacting step, or in a subsequent step, it may be preferred to use mechanical agitation to promote cleaning and removal of the broken-down soil by-products from the surface. The wash liquor preferably has a pH of from about 7 or 8 to about 11.5. The composition may typically be employed at concentrations of from about 500 ppm to about 15,000 ppm in solution to form the wash liquor. The wash liquor preferably has a temperature from about 5°C to about 40°C, or preferably from 10 to 35°C or 30°C or 25°C. When the surface is a fabric, the water to fabric ratio is typically from about 1:1 to about 30:1.

EXAMPLES

Example 1 - The binding of hemin by a binding protein within the scope of the invention (SEQ ID NO: 1) was measured using UV-vis spectroscopy according to Gao JL, Nguyen KA, Hunter N. (2010), Characterization of a hemophore-like protein from *Porphyromonas gingivalis.* J Biol Chem. 285(51):40028-38.

1:1 molar ratio of SEQ ID NO:1 and Hemin (Sigma Aldrich, product code H9039, batch H9039-1G) was incubated for 20 min at ambient room temperature before a spectral scan was performed between absorbance wavelength 300nm to 600nm, in a SpectraMax M2E plate reader.

Porphyrin binding is represented by a spectral shift in λmax, when comparing profiles of a sample containing porphyrin and a porphyrin binding protein (i.e., SEQ ID NO: 1) with a sample containing a porphyrin and nil protein.

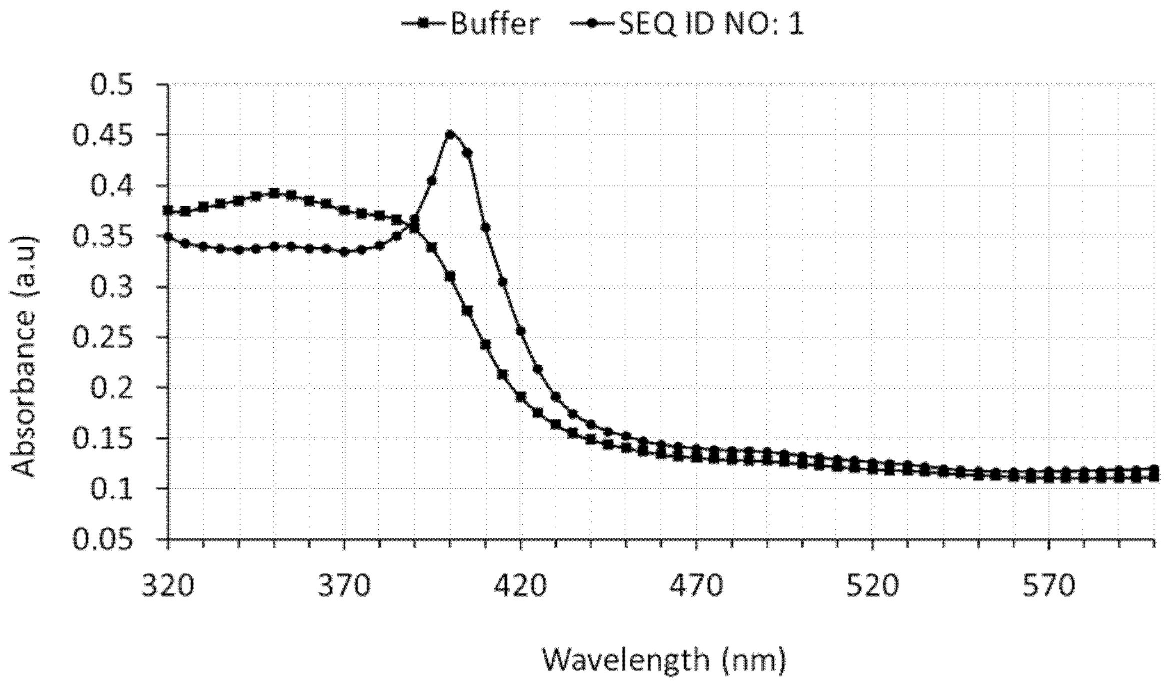


As it can be seen from the graph above, a sample containing a porphyrin such as hemin, when contacted with a porphyrin binding protein, results in a shift of $\lambda_{max}$ between 390-410 nm (solid circles), compared to a sample containing a porphyrin and nil porphyrin binding protein (solid squares). Binding of porphyrin by a porphyrin binding protein can herein be ascertained by such a shift in UV-vis absorption profile compared to a sample containing unbound porphyrin.

Example 2 - Detergent containing a porphyrin binding protein within the scope of the invention (SEQ ID NO: 1) delivers improved cleaning on soiled cotton fabrics.

The removal of particulate soil from cotton fabrics contaminated with *Pseudomonas fluorescens* was evaluated using an HDL laundry detergent comprising a porphyrin binding protein according to the invention (SEQ ID NO: 1) and an HDL laundry detergent free of porphyrin binding protein.

Stains were prepared on sterile 5cm x 5cm swatches of knitted cotton supplied by Warwick Equest Ltd, Consett, U.K. Pseudomonas fluorescens were grown overnight at 35°C on Tryptic soy agar (TSA). A single colony was used to inoculate 1x Tryptic soy broth (TSB) supplemented with 1% glucose (Sigma-Aldrich, Product code C3807993, batch SLBX8840), for 48 h at 21°C, shaking. A 1: 100 dilution of this pre-culture was made into a fresh sample of 1x TSB supplemented with 1% glucose. This was spiked onto respective 5cm x 5cm swatches of knitted cotton and incubated for 6 days at 21°C, shaking.

*Pseudomonas fluorescens* inoculated fabric swatches were treated under UV for 1h before being further stained with WFK pigment soil (Testfabrics Inc., batch: 09v-348-980). The stained swatches were left to air dry for 24 hours before use, and the color of the stains before and after washing were image analyzed using a spectrophotometer (Color-Eye 7000A, Gretag Macbeth), for CIELab values.

Stain removal testing was conducted using a Tissue lyser II (Qiagen, 1213010704C) to simulate the washing of fabrics in a washing machine. 50 ml vessels were filled with 20 ml of water (20 grains per US gallon), pre-heated to 35°C and 2.69 g/l Fairy HDL non bio (LTF1:RR71-DOV2-300G-SYEX) each. Two stained swatches were added to each pot. Final concentration of 15 ppm porphyrin binding protein (SEQ ID NO: 1) was added via a pipette to each respective wash vessel. The agitation was then initiated at 20 hz for 15 minutes for the duration of the test. After 15 minutes, the wash water was drained and the fabrics briefly rinsed for 1 minute in 1L 15°C water (20 grains per US gallon), before placing the washed stain swatches flat on a rack to dry under ambient conditions.

This process was repeated three more times, resulting in a total of eight washed stains per treatment, i.e. four external replicates, each comprising two swatches of one stains. The washed stains were analysed for CIELab as before, and Stain Removal Index (SRI) calculated using the following equation:

$$SRI = \left( \frac{\Delta E_{pre-wash} - \Delta E_{post-wash}}{\Delta E_{pre-wash}} \right) x\ 100$$

Where

$$\Delta E_{pre-wash} = \sqrt{[(L_{Clean} - L_{Stained})^2 + (a_{Clean} - a_{Stained})^2 + (b_{Clean} - b_{Stained})^2]}$$

And

$$\Delta E_{post-wash} = \sqrt{[(L_{Clean} - L_{Washed})^2 + (a_{Clean} - a_{Washed})^2 + (b_{Clean} - b_{Washed})^2]}$$

Result

| Detergent composition | SRI | SE (n=4) | P-value vs nil |
|---|---|---|---|
| Fairy HDL - Nil protein | 0.85 | 9.86 | |
| Fairy HDL + SEQ ID NO: 1 | 27.48 | 5.19 | 0.04 |
| Fairy HDL + buffer | 3.68 | 8.65 | 0.835 |
| SE is the standard error. Statistical significance is determined using Student's t-test: a p value <0.05 implies a significant difference at 95% confidence level. | | | |

As it can be seen from the data above, there is a statistically significant difference (P-value = 0.04) in performance of Fairy HDL containing SEQ ID NO: 1 vs Fairy HDL nil protein. This shows that detergent compositions comprising the

porphyrin binding protein according to the invention (SEQ ID NO: 1) provides better cleaning of fabrics soiled with particulate dirt.

DETERGENT EXAMPLES

Examples 3-8. Granular laundry detergent compositions designed for hand washing or top-loading washing machines.

| | 3 (wt %) | 4 (wt %) | 5 (wt %) | 6 (wt %) | 7 (wt %) | 8 (wt %) |
|---|---|---|---|---|---|---|
| Linear alkylbenzenesulfonate | 20 | 22 | 20 | 15 | 20 | 20 |
| C12-14 Dimethylhydroxyethyl ammonium chloride | 0.7 | 0.2 | 1 | 0.6 | 0.0 | 0.0 |
| AE3S | 0.9 | 1 | 0.9 | 0.0 | 0.5 | 0.9 |
| AE7 | 0.0 | 0.0 | 0.0 | 1 | 0.0 | 3 |
| Sodium tripolyphosphate | 5 | 0.0 | 4 | 9 | 2 | 0.0 |
| Zeolite A | 0.0 | 1 | 0.0 | 1 | 4 | 1 |
| 1.6R Silicate ($SiO_2$:$Na_2O$ at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer[1] | 0.1 | 0.2 | 0.0 | 0.0 | 0.0 | 0.0 |
| Carboxymethyl cellulose | 1 | 0.3 | 1 | 1 | 1 | 1 |
| Protease (Savinase®, 32.89 mg active/g) | 0.1 | 0.1 | 0.1 | 0.1 | | 0.1 |
| [5]DNase as defined herein (mg active per 100g composition) | 2.0 | 0 | 4.4 | 0 | 0.4 | 0 |
| Lipase - Lipex® (18 mg active /g) | 0.03 | 0.07 | 0.3 | 0.1 | 0.07 | 0.4 |
| [4]Amylase Stainzyme® Plus (mg active) | 3.0 | 5.0 | 3.0 | 2.2 | 6.0 | 6.0 |
| [6]Alginate lyase as defined herein | 12.0 | 15.0 | 3.2 | 4.3 | 9.2 | 17.0 |
| (mg active per 100g of detergent) | | | | | | |
| [7]Hexosaminidase as defined herein (mg active per 100g composition) | 2.0 | 6.0 | 5.6 | 2.2 | 4.0 | 1.5 |
| [8]Pel-ase as defined herein (mg active per 100g composition) | 4.3 | 0.0 | 3.4 | 7.2 | 1.9 | 3.3 |
| [9]Psl-ase as defined herein (mg active per 100g composition) | 3.2 | 3.2 | 0.0 | 1.2 | 0.0 | 5.3 |
| [10]Endo-beta-1,3(4)-glucanase as defined herein (mg active per 100g composition) | 9.1 | 5.4 | 0.0 | 4.3 | 3.0 | 3.1 |
| [11]Porphyrin binding protein of SEQ ID NO: 1-23 (mg active per 100g composition) | 30 | 10 | 216 | 0 | 159 | 4.2 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| $MgSO_4$ | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrate | 4.4 | 0.0 | 3.85 | 2.09 | 0.78 | 3.63 |
| NOBS | 1.9 | 0.0 | 1.66 | 0.0 | 0.33 | 0.75 |

(continued)

| | 3 (wt %) | 4 (wt %) | 5 (wt %) | 6 (wt %) | 7 (wt %) | 8 (wt %) |
|---|---|---|---|---|---|---|
| TAED | 0.58 | 1.2 | 0.51 | 0.0 | 0.015 | 0.28 |
| Sulphonated zinc phthalocyanine | 0.0030 | 0.0 | 0.0012 | 0.0030 | 0.0021 | 0.0 |
| S-ACMC | 0.1 | 0.0 | 0.0 | 0.0 | 0.06 | 0.0 |
| Direct Violet 9 | 0.0 | 0.0 | 0.0003 | 0.0005 | 0.0003 | 0.0 |
| Acid Blue 29 | 0.0 | 0.0 | 0.0 | 0.0 | 0.0 | 0.0003 |
| Sulfate/Moisture | Balance | | | | | |

Examples 9-15. Granular laundry detergent compositions designed for front-loading automatic washing machines.

| | 9 (wt%) | 10 (wt%) | 11 (wt%) | 12 (wt%) | 13 (wt%) | 14 (wt%) | 15 (wt%) |
|---|---|---|---|---|---|---|---|
| Linear alkylbenzenesulfonate | 8 | 7.1 | 7 | 6.5 | 7.5 | 7.5 | 11 |
| AE3S | 0 | 4.8 | 0 | 5.2 | 4 | 4 | 0 |
| C12-14 Alkylsulfate | 1 | 0 | 1 | 0 | 0 | 0 | 1 |
| AE7 | 2.2 | 0 | 3.2 | 0 | 0 | 0 | 1 |
| C10-12 Dimethyl hydroxyethylammonium chloride | 0.75 | 0.94 | 0.98 | 0.98 | 0 | 0 | 0 |
| Crystalline layered silicate (δ-Na2-Si2O5) | 4.1 | 0 | 4.8 | 0 | 0 | 0 | 7 |
| Zeolite A | 5 | 0 | 5 | 0 | 2 | 2 | 4 |
| Citric Acid | 3 | 5 | 3 | 4 | 2.5 | 3 | 0.5 |
| Sodium Carbonate | 15 | 20 | 14 | 20 | 23 | 23 | 14 |
| Silicate 2R (SiO2:Na2O at ratio 2:1) | 0.08 | 0 | 0.11 | 0 | 0 | 0 | 0.01 |
| Soil release agent | 0.75 | 0.72 | 0.71 | 0.72 | 0 | 0 | 0.1 |
| Acrylic Acid/Maleic Acid Copolymer | 1.1 | 3.7 | 1.0 | 3.7 | 2.6 | 3.8 | 2 |
| Carboxymethylcellulose | 0.15 | 1.4 | 0.2 | 1.4 | 1 | 0.5 | 0.2 |
| Protease - Purafect® (84 mg active/g) | 0.2 | 0.2 | 0.3 | 0.15 | 0.12 | 0.13 | 0.18 |
| Lipase - Lipex® (18.00 mg active/g) | 0.05 | 0.15 | 0.1 | 0 | 0 | 0 | 0.1 |
| Cellulase - CellucleanTM (15.6 mg active/g) | 0 | 0 | 0 | 0 | 0.1 | 0.1 | 0 |
| [4]Amylase Stainzyme® Plus (mg active) | 4.0 | 5.0 | 10 | 2.2 | 4.4 | 1.5 | 1.5 |
| Mannanase - Mannaway® (4mg active/g) | 0.05 | 0.1 | 0 | 0.05 | 0.1 | 0 | 0.1 |
| [5]DNase as defined herein (mg active per 100g detergent) | 1.0 | 0 | 2.0 | 0 | 4.0 | 0 | 0 |
| [6]Alginate lyase as defined herein (mg active per 100g of detergent) | 3.3 | 9.2 | 12.0 | 4.7 | 3.7 | 13.2 | 3.3 |
| [7]Hexosaminidase as defined herein (mg active per 100g detergent) | 3.0 | 5.0 | 8.0 | 2.2 | 4.0 | 1.5 | 0.1 |
| [8]Pel-ase as defined herein (mg active | 3.2 | 5.3 | 0.0 | 0.0 | 0.0 | 3.0 | 3.3 |

(continued)

| | 9 (wt%) | 10 (wt%) | 11 (wt%) | 12 (wt%) | 13 (wt%) | 14 (wt%) | 15 (wt%) |
|---|---|---|---|---|---|---|---|
| per 100g composition) | | | | | | | |
| [9]Psl-ase as defined herein (mg active per 100g composition) | 0.0 | 0.0 | 0.0 | 3.2 | 8.2 | 1.1 | 0.9 |
| [10]Endo-beta-1,3(4)-glucanase as defined herein (mg active per 100g composition) | 0.0 | 0.0 | 3.2 | 1.0 | 3.3 | 2.2 | 3.2 |
| [11]Porphyrin binding protein of SEQ ID NO: 1-23 (mg active per 100g composition) | 30 | 17 | 216 | 0 | 159 | 3.2 | 56 |
| TAED | 3.6 | 4.0 | 3.6 | 4.0 | 2.2 | 1.4 | 1 |
| Percarbonate | 13 | 13.2 | 13 | 13.2 | 16 | 14 | 10 |
| Na salt of Ethylenediamine-N,N'-disuccinic acid, (S,S) isomer (EDDS) | 0.2 | 0.2 | 0.001 | 0.2 | 0.2 | 0.2 | 0.001 |
| Hydroxyethane di phosphonate (HEDP) | 0.2 | 0.2 | 0.5 | 0.2 | 0.2 | 0.2 | 0.5 |
| MgSO4 | 0.42 | 0.42 | 0.42 | 0.42 | 0.4 | 0.4 | 0 |
| Perfume | 0.5 | 0.6 | 0.5 | 0.6 | 0.6 | 0.6 | 0.8 |
| Suds suppressor agglomerate | 0.05 | 0.1 | 0.05 | 0.1 | 0.06 | 0.05 | 0.05 |
| Soap | 0.45 | 0.45 | 0.45 | 0.45 | 0 | 0 | 0 |
| Sulphonated zinc phthalocyanine (active) | 0.000 7 | 0.001 2 | 0.000 7 | 0 | 0 | 0 | 0 |
| S-ACMC | 0.01 | 0.01 | 0 | 0.01 | 0 | 0 | 0 |
| Direct Violet 9 (active) | 0 | 0 | 0.000 1 | 0.000 1 | 0 | 0 | 0.001 |
| Sulfate/ Water & Miscellaneous | Balance | | | | | | |
| *DNase is shown as mgs of active enzyme per 100g of detergent. | | | | | | | |

Examples 16-23. Heavy Duty Liquid laundry detergent compositions

| | 16 (wt%) | 17 (wt%) | 18 (wt%) | 19 (wt%) | 20 (wt%) | 21 (wt%) | 22 (wt%) | 23 (wt%) |
|---|---|---|---|---|---|---|---|---|
| C12-15 | 14.7 | 11.6 | 0.0 | 16.3 | 0.0 | 17.3 | 20 | 12 |
| Alkylethoxy(1.8)sulfate | | | | | | | | |
| C11.8 Alkylbenzene sulfonate | 4.3 | 11.6 | 8.3 | 7.8 | 11.7 | 7.8 | 7 | 0 |
| C16-17 Branched alkyl sulfate | 1.7 | 1.29 | 0.0 | 3.09 | 0.0 | 3.3 | 0 | 0 |
| C12-14 Alkyl -9-ethoxylate | 0.9 | 1.07 | 0.0 | 1.31 | 0.0 | 1.31 | 5 | 0 |
| C12 dimethylamine oxide | 0.6 | 0.64 | 0.0 | 1.03 | 0.0 | 1.03 | 2 | 3 |
| Citric acid | 3.5 | 0.65 | 3 | 0.66 | 2.27 | 0.67 | 1 | 0 |
| C12-18 fatty acid | 1.5 | 2.32 | 3.6 | 1.52 | 0.82 | 1.52 | 1 | 0 |
| Sodium Borate (Borax) | 2.5 | 2.46 | 1.2 | 2.53 | 0.0 | 2.53 | 0 | 1 |
| Sodium C12-14 alkyl ethoxy 3 sulfate | 0.0 | 0.0 | 2.9 | 0.0 | 3.9 | 0.0 | 0 | 14 |

(continued)

| | 16 (wt%) | 17 (wt%) | 18 (wt%) | 19 (wt%) | 20 (wt%) | 21 (wt%) | 22 (wt%) | 23 (wt%) |
|---|---|---|---|---|---|---|---|---|
| C14-15 alkyl 7-ethoxylate | 0.0 | 0.0 | 4.2 | 0.0 | 1.9 | 0.0 | 0 | 4.2 |
| C12-14 Alkyl -7-ethoxylate | 0.0 | 0.0 | 1.7 | 0.0 | 0.5 | 0.0 | 0 | 1.7 |
| Ca chloride dihydrate | 0.0 | 0.0 | 0.0 | 0.0 | 0.045 | 0.0 | 0 | 0 |
| Ca formate | 0.09 | 0.09 | 0.0 | 0.09 | 0.0 | 0.09 | 0.09 | 0 |
| A compound: bis((C2H5O)(C2H4O)n)(C H3)-N+-CxH2x-N+-(CH3)-bis((C2H5O)(C2H4O)n); n is 20 to 30; x is 3 to 8, optionally sulphated or sulphonated | 0.0 | 0.0 | 1.2 | 0.0 | 0.66 | 0.0 | 0.0 | 1.2 |
| Random graft co-polymer[1] | 0.0 | 1.46 | 0.5 | 0.0 | 0.83 | 0.0 | 0.0 | 0.5 |
| Ethoxylated Polyethylenimine [2] | 1.5 | 1.29 | 0.0 | 1.44 | 0.0 | 1.44 | 1.44 | 0.0 |
| Diethylene triamine pentaacetic acid | 0.34 | 0.64 | 0.0 | 0.34 | 0.0 | 0.34 | 0.34 | 0.0 |
| Diethylene triamine penta | 0.0 | 0.0 | 0.3 | 0.0 | 0.3 | 0.0 | 0.0 | 0.3 |
| (methylene phosphonic acid) | | | | | | | | |
| 1-hydroxyethyidene-1,1-diphosphonic acid | 0.0 | 0.0 | 0.0 | 0.0 | 0.18 | 0.0 | 0.0 | 0.0 |
| Dihydroxybenzene-3,5-disulfonic acid disodium salt hydrate | 0.0 | 0.0 | 0.0 | 0.0 | 0.0 | 0.19 | 0.19 | 0.0 |
| Tinopal AMS-GX | 0.0 | 0.06 | 0.0 | 0.0 | 0.0 | 0.29 | 0.29 | 0.0 |
| Tinopal CBS-X | 0.2 | 0.17 | 0.0 | 0.29 | 0.0 | 0.0 | 0.0 | 0.0 |
| Tinopal TAS-X B36 | 0.0 | 0.0 | 0.0 | 0.0 | 0.091 | 0.0 | 0.0 | 0.0 |
| Amphiphilic alkoxylated grease cleaning polymer [3] | 1.28 | 1 | 0.4 | 1.93 | 0.0 | 1.93 | 1.93 | 0.4 |
| CHEC | 0.0 | 0.0 | 0.2 | 0.0 | 0.0 | 0.0 | 0.0 | 0.2 |
| Ethanol | 2 | 1.58 | 1.6 | 5.4 | 1.2 | 3.57 | 0 | 1.6 |
| Propylene Glycol | 3.9 | 3.59 | 1.3 | 4.3 | 0.0 | 3.8 | 3.8 | 1.3 |
| Diethylene glycol | 1.05 | 1.54 | 0.0 | 1.15 | 0.0 | 1.15 | 1.15 | 0.0 |
| Polyethylene glycol | 0.06 | 0.04 | 0.0 | 0.1 | 0.0 | 0.1 | 0.1 | 0.0 |
| [4]Amylase Amplify® (mg active) | 8.0 | 7.0 | 2.5 | 4.0 | 3.0 | 1.7 | 3 | 2.5 |
| [5]DNase (mg active per 100g detergent) | 0 | 2.0 | 0 | 2.0 | 1.25 | 1.0 | 5 | 0 |
| [6]Alginate lyase as defined herein (mg active per 100g of detergent) | 3.2 | 4.1 | 7.9 | 12.4 | 3.7 | 5.0 | 17.3 | 2.1 |
| [7]Hexosaminidase as defined herein (mg active per 100g detergent) | 7.0 | 3.0 | 8.0 | 2.2 | 1.25 | 10 | 0.1 | 2.5 |
| [8]Pel-ase as defined herein (mg active per 100g composition) | 2.2 | 0.0 | 1.2 | 0.8 | 0.10 | 8.3 | 2.2 | 3.9 |
| [9]Psl-ase as defined herein (mg active per 100g | 3.4 | 4.4 | 0.0 | 0.0 | 0.0 | 4.3 | 5.3 | 3.2 |

(continued)

| | 16 (wt%) | 17 (wt%) | 18 (wt%) | 19 (wt%) | 20 (wt%) | 21 (wt%) | 22 (wt%) | 23 (wt%) |
|---|---|---|---|---|---|---|---|---|
| composition) | | | | | | | | |
| [10]Endo-beta-1,3(4)-glucanase as defined herein (mg active per 100g composition) | 2.2 | 0.0 | 1.4 | 5.3 | 2.2 | 3.3 | 4.5 | 3.3 |
| [11]Porphyrin binding protein of SEQ ID NO: 1-23 (mg active per 100g composition) | 30 | 17 | 216 | 0 | 159 | 3.2 | 56 | 18.2 |
| Monoethanolamine | 3.05 | 2.41 | 0.4 | 1.26 | 0.31 | 1.13 | 1.13 | 0.4 |
| NaOH | 2.44 | 1.8 | 0.0 | 3.01 | 3.84 | 0.24 | 0.24 | 0.0 |
| Sodium Cumene Sulphonate | 0.0 | 0.0 | 1 | 0.0 | 0.95 | 0.0 | 0.0 | 1 |
| Sodium Formate | 0.0 | 0.11 | 0.0 | 0.09 | 0.2 | 0.12 | 0.12 | 0.0 |
| Polyethoxylated azo thiophene dye | 0.001 | 0.001 | 0.001 | 0.05 | 0.000 1 | 0.000 1 | 0.000 1 | 0.001 |
| Water, Aesthetics (Dyes, perfumes) and Minors (Enzymes including lipase, protease, additional amylase each at 0.2% active protein, solvents, structurants) | balance | | | | | | | |

Examples 24-30. Unit Dose Laundry detergent compositions. Such unit dose formulations can comprise one or multiple compartments.

| | 24 (wt%) | 25 (wt%) | 26 (wt%) | 27 (wt%) | 28 (wt%) | 29 (wt%) | 30 (wt%) |
|---|---|---|---|---|---|---|---|
| Alkylbenzene sulfonic acid | 14.5 | 14.5 | 14.5 | 14.5 | 14.5 | 23 | 23 |
| C12-18 alkyl ethoxy 2.5 sulfate | 7.5 | 7.5 | 7.5 | 7.5 | 7.5 | 16 | 16 |
| C12-18 alkyl 7-ethoxylate | 13.0 | 13.0 | 13.0 | 13.0 | 13.0 | 3.1 | 3.8 |
| C14-15 alkyl 9-ethoxylate | 0 | 0 | 0 | 0 | 0 | 1 | 0 |
| Citric Acid | 0.6 | 0.6 | 0.6 | 0.6 | 0.6 | 0.9 | 0.7 |
| Fatty Acid | 14.8 | 14.8 | 14.8 | 14.8 | 14.8 | 6.5 | 6 |
| Amylase (mg active) | 6 | 12 | 8 | 2 | 10 | 2 | 2 |
| Ethoxylated Polyethylenimine[2] | 4.0 | 4.0 | 4.0 | 4.0 | 4.0 | 4.0 | 4.0 |
| Protease (Purafect Prime®, 40.6 mg active/g) | 1.4 | 2.0 | 0.9 | 1.2 | 0 | 1 | 1 |
| Cellulase (Celluclean, active protein) | 0.1 | 0.2 | 0.0 | 0.0 | 0.1 | 0 | 0 |
| [5]DNase described herein (mg active per 100g detergent) | 3.0 | 2.0 | 1.0 | 2.0 | 2.0 | 1 | 1 |
| [4]Amylase Amplify® (active protein) | 0.0 | 0.0 | 0.1 | 0.2 | 0.1 | 0.5 | 0.5 |
| [6]Alginate lyase as defined herein (mg active per 100g of detergent) | 2.2 | 3.1 | 2.3 | 5.2 | 5.3 | 12.2 | 5.4 |

(continued)

| | 24 (wt%) | 25 (wt%) | 26 (wt%) | 27 (wt%) | 28 (wt%) | 29 (wt%) | 30 (wt%) |
|---|---|---|---|---|---|---|---|
| [7]Hexosaminidase as defined herein (mg active per 100g detergent) | 5.0 | 10 | 0.0 | 2.2 | 1.2 | 3 | 0.9 |
| [8]Pel-ase as defined herein (mg active per 100g composition) | 1.3 | 0.0 | 2.45 | 8.3 | 4.0 | 3.2 | 5.3 |
| "Psi-ase as defined herein (mg active per 100g composition) | 0.0 | 3.4 | 7.3 | 3.2 | 1.5 | 4.7 | 0.0 |
| [10]Endo-beta-1,3(4)-glucanase as defined herein (mg active per 100g composition) | 3.4 | 3.3 | 3.4 | 0.0 | 3.1 | 1.9 | 6.3 |
| [11]Porphyrin binding protein of SEQ ID NO: 1-23 (mg active per 100g composition) | 30 | 17 | 216 | 0 | 159 | 3.2 | 56 |
| Hydroxyethane diphosphonic acid | 1.2 | 1.2 | 1.2 | 1.2 | 1.2 | 0 | 2.3 |
| Brightener | 0.3 | 0.3 | 0.3 | 0.3 | 0.3 | 0.2 | 0.2 |
| P-diol | 15.8 | 13.8 | 13.8 | 13.8 | 13.8 | 12.2 | 12.2 |
| Glycerol | 6.1 | 6.1 | 6.1 | 6.1 | 6.1 | 4.0 | 3.8 |
| MEA | 8.0 | 8.0 | 8.0 | 8.0 | 8.0 | 8.6 | 10.2 |
| TIPA | 0.0 | 0.0 | 2.0 | 0.0 | 0.0 | 0.0 | 0.0 |
| TEA | 0.0 | 2.0 | 0.0 | 0.0 | 0.0 | 0.0 | 0.0 |
| Cumene sulphonate | 0.0 | 0.0 | 0.0 | 0.0 | 2.0 | 0.0 | 0.0 |
| Cyclohexyl dimethanol | 0.0 | 0.0 | 0.0 | 2.0 | 0.0 | 0.0 | 0.0 |
| Water | 10 | 10 | 10 | 10 | 10 | 10 | 10 |
| Structurant | 0.14 | 0.14 | 0.14 | 0.14 | 0.14 | 0.14 | 0.14 |
| Perfume | 1.9 | 1.9 | 1.9 | 1.9 | 1.9 | 1.9 | 1.9 |
| Buffers (monoethanolamine) | To pH 8.0 | | | | | | |
| Solvents (1,2 propanediol, ethanol) & minors | To 100% | | | | | | |

Example 31. Multiple Compartment Unit Dose Composition

Multiple compartment unit dose laundry detergent formulations of the present invention are provided below. In these examples the unit dose has three compartments, but similar compositions can be made with two, four or five compartments. The film used to encapsulate the compartments is polyvinyl alcohol.

| Base composition 1 | 31 (wt%) |
|---|---|
| Glycerol (min 99) | 5.3 |
| 1,2-propanediol | 10.0 |
| Citric Acid | 0.5 |
| Monoethanolamine | 10.0 |
| Caustic soda | - |
| Dequest 2010 | 1.1 |
| Potassium sulfite | 0.2 |
| [5]DNase as defined herein (mg active) | 4.0 |
| [6]Alginate lyase as defined herein (mg active per 100g of detergent) | 12.2 |
| [7]Hexosaminidase as defined herein (mg active per 100g detergent) | 7.0 |

(continued)

| Base composition 1 | 31 (wt%) |
|---|---|
| [8]Pel-ase as defined herein (mg active per 100g composition) | 2.3 |
| [9]Psl-ase as defined herein (mg active per 100g composition) | 4.2 |
| [10]Endo-beta-1,3(4)-glucanase as defined herein (mg active per 100g composition) | 5.4 |
| [11]Porphyrin binding protein of SEQ ID NO: 1-23 (mg active per 100g composition) | 30.2 |
| Nonionic Marlipal C24EO7 | 20.1 |
| HLAS | 24.6 |
| Optical brightener FWA49 | 0.2 |
| C12-15 Fatty acid | 16.4 |
| Polymer Lutensit Z96 | 2.9 |
| Polyethyleneimine ethoxylate PEI600 E20 | 1.1 |
| MgCl2 | 0.2 |
| Solvents (1,2 propanediol, ethanol) | To 100% |

Multi-compartment formulations

| Composition | 1 | | | 2 | | |
|---|---|---|---|---|---|---|
| Compartment | A | B | C | A | B | C |
| Volume of each compartment | 40 ml | 5 ml | 5 ml | 40 ml | 5 ml | 5 ml |
| Active material in Wt.% | | | | | | |
| Perfume | 1.6 | 1.6 | 1.6 | 1.6 | 1.6 | 1.6 |
| Dyes | < 0.01 | < 0.01 | < 0.01 | < 0.01 | < 0.01 | < 0.01 |
| TiO2 | 0.1 | - | - | - | 0.1 | - |
| Sodium Sulfite | 0.4 | 0.4 | 0.4 | 0.3 | 0.3 | 0.3 |
| Acusol 305 | 1.2 | | | 2 | - | - |
| Hydrogenated castor oil | 0.14 | 0.14 | 0.14 | 0.14 | 0.14 | 0.14 |
| Base Composition 1 | Add to 100% | Add to 100% | Add to 100% | Add to 100% | Add to 100% | Add to 100% |

Raw Materials and Notes for Composition Examples 3-31

Linear alkylbenzenesulfonate having an average aliphatic carbon chain length C11-C18

C12-18 Dimethylhydroxyethyl ammonium chloride
AE3S is C12-15 alkyl ethoxy (3) sulfate
AE7 is C12-15 alcohol ethoxylate, with an average degree of ethoxylation of 7
AE9 is C12-16 alcohol ethoxylate, with an average degree of ethoxylation of 9
HSAS is a mid-branched primary alkyl sulfate with carbon chain length of about 16-17 as disclosed in US 6,020,303 and US 6,060,443
Polyacrylate MW 4500 is supplied by BASF
Carboxymethyl cellulose is Finnfix® V supplied by CP Kelco, Arnhem, Netherlands
CHEC is a cationically modified hydroxyethyl cellulose polymer.
Phosphonate chelants are, for example, diethylenetetraamine pentaacetic acid (DTPA) Hydroxyethane di phosphonate (HEDP)
Savinase®, Natalase®, Stainzyme®, Lipex®, CellucleanTM, Mannaway® and Whitezyme® are all products of Novozymes, Bagsvaerd, Denmark.
Purafect®, Purafect Prime® are products of Genencor International, Palo Alto, California, USA
Fluorescent Brightener 1 is Tinopal® AMS, Fluorescent Brightener 2 is Tinopal® CBS-X, Direct Violet 9 is Pergasol® Violet BN-Z NOBS is sodium nonanoyloxybenzenesulfonate TAED is tetraacetylethylenediamine
S-ACMC is carboxymethylcellulose conjugated with C.I. Reactive Blue 19product name AZO-CM-CELLULOSE
Soil release agent is Repel-o-tex® PF
Acrylic Acid/Maleic Acid Copolymer is molecular weight 70,000 and acrylate:maleate ratio 70:30
EDDS is a sodium salt of ethylenediamine-N,N'-disuccinic acid, (S,S) isomer Suds suppressor agglomerate is supplied by Dow Corning, Midland, Michigan, USA
HSAS is mid-branched alkyl sulfate
Liquitint® Violet CT polymeric hueing dye, supplied by Milliken, Spartanburg, South Carolina, USA
Polyethoxylated azo thiophene dye is Violet DD™ polymeric hueing dye, supplied by Milliken, Spartanburg, South Carolina, USA.
[1] Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.
[2] Polyethyleneimine (MW = 600) with 20 ethoxylate groups per -NH.
[3] Amphiphilic alkoxylated polymer is a polyethylenimine (MW 600), prepared from a polymer that is derivatised to contain 24 ethoxylate groups per -NH and 16 Propoxylate groups per -NH.
[4]Amylase is shown as mgs of active enzyme per 100g of detergent.
[5]DNase as described herein (in all of these examples is shown as mgs of active enzyme per 100g of detergent). DNase may comprise minor amounts of super oxide dismutase impurity.
[6]Alginate lyase as described herein (in all examples shown as mgs of active enzyme per 100g of detergent)
[7]Hexosaminidase as described herein (in all examples shown as mgs of active enzyme per 100g of detergent)
[8]Pel-ase as described herein (in all examples shown as mgs of active enzyme per 100g of detergent)
[9]Psl-ase as described herein (in all examples shown as mgs of active enzyme per 100g of detergent)
[10]Endo-beta-1,3(4)-glucanase as described herein (in all examples shown as mgs of active enzyme per 100g of detergent).[a] Proxel GXL, 20% aqueous dipropylene glycol solution of 1,2-benzisothiazolin-3-one, supplied by Lonza.
[11]Porphyrin binding protein of SEQ ID NO: 1-23 (in all examples shown as mg of active enzyme per 100g of composition)
[b] N,N-bis(hydroxyethyl)-N,N-dimethyl ammonium chloride fatty acid ester. The iodine value of the parent fatty acid of this material is between 18 and 22. The material as obtained from Evonik contains impurities in the form of free fatty acid, the monoester form of N,N-bis(hydroxyethyl)-N,N-dimethyl ammonium chloride fatty acid ester, and fatty acid esters of N,N-bis(hydroxyethyl)-N-methylamine.
[c] MP10®, supplied by Dow Corning, 8% activity
[d] as described in US 8,765,659, expressed as 100% encapsulated perfume oil
[e] Rheovis® CDE, cationic polymeric thickener supplied by BASF
[f] N,N-dimethyl octanamide and N,N-dimethyl decanamide in about a 55:45 weight ratio, tradename Steposol® M-8-10 from the Stepan Company

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

**1.** A treatment composition comprising:

a) from 0.00005 to 5% by weight thereof of a porphyrin binding protein; and
b) at least one adjunct component preferably selected from a surfactant, a builder, a bleach component, a polymer, a dispersing agent and an enzyme.

**2.** A treatment composition according to claim 1 wherein the porphyrin binding protein is selected from the group of

consisting of HusA, HxuA, IsdH/NEAT, HmuY, GADPH, Hemophilin, Hbp2, HphA, HfpY, HasA and mixtures thereof.

3. A treatment composition according to claim 1 wherein the porphyrin binding protein is selected from the group of porphyrin binding proteins having at least 35% sequence identity to any of sequences SEQ ID NO: 1 to SEQ ID NO: 23.

4. A treatment composition according to the preceding claim wherein the porphyrin binding protein is selected from the group of porphyrin binding proteins having at least 45%, preferably at least 65%, preferably at least 75%, preferably at least 85%, preferably at least 95%, preferably at least 98%, preferably at least 99%, preferably 100% sequence identity to any of sequences SEQ ID NO: 1 to SEQ ID NO: 23.

5. A treatment composition according to the preceding claim wherein the porphyrin binding protein is selected from the group of porphyrin binding proteins having 100% sequence identity to any of sequences SEQ ID NO: 1 to SEQ ID NO: 23.

6. A treatment composition according to claim 1 wherein the porphyrin binding protein is selected from the group of porphyrin binding proteins having a Z-score, as calculated using the Distance matrix alignment algorithm, of at least 4 versus PDB ref: 6BQS, PDB ref: 6U87, Uniprot accession A2BCY8, PDB ref 5M6D, Uniprot accession Q8NW39, Uniprot accession P44602, Uniprot accession I6X8R5, Uniprot accession A0A502JZY8 or Uniprot accession Q7AP54.

7. A treatment composition according to the preceding claim wherein the porphyrin binding protein is selected from the group of porphyrin binding proteins having a Z-score, as calculated using the Distance matrix alignment algorithm, of at least 5, preferably at least 6, preferably at least 7, preferably at least 8, preferably at least 9, preferably at least 10, preferably at least 11, preferably at least 12, preferably at least 13, preferably at least 14, preferably at least 15 versus PDB ref 6BQS, PDB ref 6U87, Uniprot accession A2BCY8, PDB ref 5M6D, Uniprot accession Q8NW39, Uniprot accession P44602, Uniprot accession I6X8R5, Uniprot accession A0A502JZY8 or Uniprot accession Q7AP54.

8. A treatment composition according to any of the preceding claims wherein the composition comprises an enzyme, preferably an amylase and a protease.

9. A treatment composition according to any of the preceding claims wherein the composition comprises a surfactant.

10. A treatment composition according to any of the preceding claims wherein the composition is a laundry detergent composition.

11. A treatment composition according to any of the preceding claims wherein the composition has a pH of greater than 7.5, preferably from 8 to 11.5, as measured in a 1% by weight aqueous solution at 25°C.

12. A treatment composition according to any of the preceding claims wherein the composition is a laundry composition comprising:

a) from about to about 1 to about 40% by weight of the composition of a surfactant, preferably an anionic surfactant;
b) from 0.00005 to 5% by weight of the composition of an enzyme comprising an amylase and a protease and optionally a lipase;
c) from about 10 to about 60% by weight of the composition of a builder, preferably a builder selected from builders based on carbonate, bicarbonate or silicates; and
d) optionally a soil release polymer.

13. A treatment composition according to any of the preceding claims wherein the composition is an automatic dishwashing composition comprising:

a) from about 1 to about 10% by weight of the composition of a surfactant, preferably non-nionic surfactant;
b) from 0.00005 to 5% by weight of the composition of an enzyme comprising an amylase and a protease;
c) from about 5 to about 50% by weight of the composition of a builder, preferably selected from citrate, a salt of methyl glycine diacetic acid and a mixture thereof; and
d) optionally a polymer, preferably a sulfonate polymer.

**14.** A method of treating a substrate comprising the step of contacting the substrate with a composition according to any of the preceding claims and optionally rinsing the substrate.

**15.** Use of a porphyrin binding protein in a treatment composition to provide soil removal.

**16.** Use of a porphyrin binding protein in a laundry treatment composition to provide soil removal from fabrics.

Europäisches Patentamt
European Patent Office
Office européen des brevets

# EUROPEAN SEARCH REPORT

Application Number
EP 23 18 3075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2019 106038 A1 (HENKEL AG & CO KGAA [DE]) 10 September 2020 (2020-09-10)<br>* paragraph [0001] - paragraph [0006] *<br>* paragraph [0008] *<br>* paragraph [0015] - paragraph [0017] *<br>* paragraph [0036] - paragraph [0038] *<br>* paragraph [0041] - paragraph [0042] *<br>* paragraph [0044] - paragraph [0066] *<br>* paragraph [0075] - paragraph [0078] *<br>* paragraph [0087] - paragraph [0089] *<br>* paragraph [0095] - paragraph [0099] *<br>* paragraph [0119] - paragraph [0122]; examples; table 1 *<br>* claims 1-12 *<br>----- | 1,8-16 | INV.<br>C11D3/38<br>C11D11/00<br>C11D1/02<br>C11D3/386<br>C07K14/195 |
| X | US 2011/071067 A1 (REINHARDT GERD [DE] ET AL) 24 March 2011 (2011-03-24)<br>* paragraphs [0001] - [0003], [0005], [0006], [0010] - [0033], [0037], [0038], [0049]; claims; examples *<br>----- | 1,6-16 | |
| | | | TECHNICAL FIELDS SEARCHED (IPC) |
| X | US 2003/008794 A1 (JAYNES BINGHAM SCOTT [US]) 9 January 2003 (2003-01-09)<br>* claims 1-4 *<br>* paragraph [0053] - paragraph [0055]; example 4 *<br>----- | 1,6,7, 11,14-16 | C11D<br>C12R<br>C07K |
| X | US 2015/291943 A1 (KIEMER LARS [DK] ET AL) 15 October 2015 (2015-10-15)<br>* paragraphs [0003] - [0009], [0098] - [0106], [0225], [0226]; claims 30-39; examples *<br>-----<br>-/-- | 1,6-16 | |

The present search report has been drawn up for all claims

| Place of search | Date of completion of the search | Examiner |
|---|---|---|
| The Hague | 16 November 2023 | Marttin, Emmeline |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

page 1 of 2

Europäisches Patentamt
European Patent Office
Office européen des brevets

# EUROPEAN SEARCH REPORT

Application Number
EP 23 18 3075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 171 345 B1 (CONVENTS DANIEL [BE] ET AL) 9 January 2001 (2001-01-09)<br>* column 1, paragraph 2-8 *<br>* column 4, last paragraph - paragraph 1 *<br>* column 4, line 61 - column 5, line 4 *<br>* column 5, line 35 - column 6, line 59; claims; examples *<br>----- | 1,2,6-16 | |
| X | JP 6 269946 B2 (UNIV NAGOYA)<br>31 January 2018 (2018-01-31)<br>* paragraph [0004]; claims 1-5 *<br>----- | 1-7,14 | |
| X | WO 2020/174044 A1 (EVAXION BIOTECH APS [DK]) 3 September 2020 (2020-09-03)<br>* claims 1,62; sequence 24 *<br>* paragraph [0852] - paragraph [0864] *<br>----- | 1-7,14 | |
| X | US 2022/396601 A1 (ZHANG LI [US])<br>15 December 2022 (2022-12-15)<br>* paragraph [0006]; claims 21,31,35,39,44-45 *<br>----- | 1-4,6,7,14 | TECHNICAL FIELDS SEARCHED (IPC) |

The present search report has been drawn up for all claims

| Place of search | Date of completion of the search | Examiner |
|---|---|---|
| The Hague | 16 November 2023 | Marttin, Emmeline |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 18 3075

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2023

| Patent document cited in search report | Publication date | Patent family member(s) | Publication date |
|---|---|---|---|
| DE 102019106038 A1 | 10-09-2020 | NONE | |
| US 2011071067 A1 | 24-03-2011 | BR PI0912764 A2 | 23-05-2017 |
| | | CN 102159697 A | 17-08-2011 |
| | | DE 102008024084 A1 | 19-11-2009 |
| | | EP 2300587 A1 | 30-03-2011 |
| | | ES 2417754 T3 | 09-08-2013 |
| | | JP 2011521027 A | 21-07-2011 |
| | | KR 20110040763 A | 20-04-2011 |
| | | PL 2300587 T3 | 30-09-2013 |
| | | US 2011071067 A1 | 24-03-2011 |
| | | WO 2009141073 A1 | 26-11-2009 |
| US 2003008794 A1 | 09-01-2003 | BR 0210612 A | 28-09-2004 |
| | | CA 2450893 A1 | 09-01-2003 |
| | | EP 1399535 A1 | 24-03-2004 |
| | | JP 2004533523 A | 04-11-2004 |
| | | MX PA03011905 A | 03-06-2004 |
| | | US 2003008794 A1 | 09-01-2003 |
| | | WO 03002707 A1 | 09-01-2003 |
| US 2015291943 A1 | 15-10-2015 | CN 104837990 A | 12-08-2015 |
| | | EP 2929020 A1 | 14-10-2015 |
| | | US 2015291943 A1 | 15-10-2015 |
| | | WO 2014086928 A1 | 12-06-2014 |
| US 6171345 B1 | 09-01-2001 | AR 007719 A1 | 10-11-1999 |
| | | AU 3337997 A | 02-02-1998 |
| | | BR 9710997 A | 14-03-2000 |
| | | CA 2257221 A1 | 15-01-1998 |
| | | CN 1229427 A | 22-09-1999 |
| | | EP 0912674 A1 | 06-05-1999 |
| | | ID 17487 A | 08-01-1998 |
| | | IN 189794 B | 19-04-2003 |
| | | TR 199802781 T2 | 22-02-1999 |
| | | US 6171345 B1 | 09-01-2001 |
| | | US 6352968 B1 | 05-03-2002 |
| | | WO 9801523 A1 | 15-01-1998 |
| | | ZA 975896 B | 04-01-1999 |
| JP 6269946 B2 | 31-01-2018 | JP 6269946 B2 | 31-01-2018 |
| | | JP 2015182982 A | 22-10-2015 |
| WO 2020174044 A1 | 03-09-2020 | EP 3931206 A1 | 05-01-2022 |
| | | US 2022143168 A1 | 12-05-2022 |
| | | WO 2020174044 A1 | 03-09-2020 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 18 3075

This annex lists the patent family members relating to the patent documents cited in the above-mentioned European search report. The members are as contained in the European Patent Office EDP file on
The European Patent Office is in no way liable for these particulars which are merely given for the purpose of information.

16-11-2023

| Patent document cited in search report | Publication date | Patent family member(s) | Publication date |
|---|---|---|---|
| US 2022396601 A1 | 15-12-2022 | US 2020361999 A1 | 19-11-2020 |
| | | US 2022396601 A1 | 15-12-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004067737 A
- WO 2015091989 A
- WO 2015091990 A
- WO 2015024739 A
- WO 2015143360 A
- US 6312936 B1
- US 5679630 A
- US 4760025 A
- DE 102006022216 A1
- DE 102006022224 A1
- WO 2015089447 A
- WO 2015089441 A
- WO 2016066756 A
- WO 2016066757 A
- WO 2016069557 A
- WO 2016069563 A
- WO 2016069569 A
- WO 2016174234 A
- WO 8906270 A
- WO 05052161 A
- WO 05052146 A
- WO 07044993 A2
- WO 2014194032 A
- WO 2014194054 A
- WO 2014194117 A
- WO 2015193488 A
- WO 2016075078 A
- WO 9217577 A
- WO 0037627 A
- WO 08010925 A
- US 5352604 A
- US 6939702 B1
- US 20090217464 A
- WO 2013171241 A
- WO 2011084412 A
- WO 2013033318 A
- WO 2018228880 A
- WO 2018228881 A
- EP 3299457 A
- WO 2018209026 A
- WO 2017036901 A
- WO 2017005798 A
- US 4435307 A
- US 5648263 A
- US 5691178 A
- US 5776757 A
- US 7141403 B2
- WO 09148983 A
- WO 2017106676 A
- US 7153818 B
- WO 9700324 A
- EP 1022334 A
- US 5856164 A
- WO 9923211 A
- WO 9623873 A
- WO 0060060 A
- WO 06002643 A
- WO 2017192657 A
- WO 2011100410 A
- WO 2013003659 A
- US 6093562 A
- WO 09149130 A
- WO 10115021 A
- WO 2016091688 A
- WO 2014099523 A
- WO 2014164777 A
- WO 2009149271 A
- WO 2016180748 A
- WO 2018060216 A
- WO 9324618 A
- WO 9510602 A
- WO 9815257 A
- WO 2018191135 A
- WO 2015040159 A
- WO 2017021515 A
- WO 2017021516 A
- WO 2017021517 A
- WO 2019081515 A
- WO 2017162836 A
- WO 2018108865 A
- WO 2018011277 A
- EP 3476935 A
- WO 2018178061 A
- WO 2020074499 A
- WO 2018184873 A
- WO 2017186936 A
- WO 2017186937 A
- WO 2017186943 A
- WO 2017207770 A
- WO 2019086520 A
- WO 2019086528 A
- WO 2019086530 A
- WO 2019086532 A
- WO 2019086521 A
- WO 2019086526 A
- WO 2020002604 A
- WO 2020002608 A
- WO 2020007863 A

- WO 2020007875 A
- WO 2020008024 A
- WO 2020070063 A
- WO 2020070249 A
- WO 2020088957 A
- WO 2020088958 A
- WO 2020207944 A
- WO 2022094164 A
- WO 2019228448 A
- WO 2018102478 A
- WO 2018185181 A
- WO 2020070011 A
- WO 2018185267 A
- WO 2020070209 A
- WO 2020008043 A
- WO 2018102479 A
- WO 2018185280 A
- WO 2022094588 A
- WO 2022094163 A
- WO 2013167581 A
- WO 2015181299 A
- WO 2015181292 A
- WO 2017046232 A
- WO 2017046260 A
- WO 201837062 A
- WO 201837065 A
- WO 2019038059 A
- WO 2019162000 A
- WO 2015001017 A
- WO 2018037061 A
- WO 201837064 A
- WO 2019038060 A
- WO 2019038057 A
- WO 2015185689 A
- EP 1794275 A
- EP 1794276 A
- US 7208459 B2
- WO 201198355 A
- WO 201147987 A
- US 2012090102 A
- WO 2010145887 A
- WO 2006055787 A
- WO 2010142503 A
- US 20080177090 A
- WO 2011011799 A
- US 8138222 B
- WO 09118375 A
- WO 9813459 A
- US 6020303 A
- US 6060443 A
- US 8765659 B


**Non-patent literature cited in the description**


- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **HOLM L.** Using Dali for Protein Structure Comparison. *Methods Mol Biol.*, 2020, vol. 2112, 29-42
- **SENIOR, A.W.** ; **EVANS, R.** ; **JUMPER, J. et al.** Improved protein structure prediction using potentials from deep learning. *Nature*, 2020, vol. 577, 706-710
- **HOLM L** ; **KÄÄRIÄINEN S** ; **ROSENSTRÖM P** ; **SCHENKEL A**. Searching protein structure databases with DaliLite v.3. *Bioinformatics*, 01 December 2008, vol. 24 (23), 2780-1
- **METROPOLIS, N.** ; **ROSENBLUTH, A. W.** ; **ROSENBLUTH, M. N.** ; **TELLER, A. H.** ; **TELLER, E.** Equation of state calculations by fast computing machines. *J. Chem. Phys.*, 1953, vol. 21, 1087-1092
- **FALICOV A** ; **COHEN FE**. A surface of minimum area metric for the structural comparison of proteins. *J Mol Biol*, 1996, vol. 258, 871-892
- **HOLM L** ; **SANDER C**. Mapping the protein universe. *Science*, 1996, vol. 273, 595-602
- **BATEMAN TJ** ; **SHAH M** ; **HO TP** ; **SHIN HE** ; **PAN C** ; **HARRIS G** ; **FEGAN JE** ; **ISLAM EA** ; **AHN SK** ; **HOODA Y**. A Slam-dependent hemophore contributes to heme acquisition in the bacterial pathogen Acinetobacter baumannii. *Nat Commun.*, 01 November 2021, vol. 12 (1), 6270
- **WILSON T** ; **MOURIÑO S** ; **WILKS A.** The heme-binding protein PhuS transcriptionally regulates the Pseudomonas aeruginosa tandem sRNA prrF1,F2 locus. *J Biol Chem.*, January 2021, vol. 296, 100275
- **SHOJI M** ; **SATO K** ; **YUKITAKE H** ; **KONDO Y** ; **NARITA Y et al.** Por Secretion System-Dependent Secretion and Glycosylation of Porphyromonas gingivalis Hemin-Binding Protein 35. *PLOS ONE*, 2018, vol. 13 (8), e0203154
- **GRIGG JC** ; **MAO CX** ; **MURPHY ME**. Iron-coordinating tyrosine is a key determinant of NEAT domain heme transfer. *J Mol Biol.*, vol. 413 (3), 684-98
- **HOFBAUER S** ; **PFANZAGL V** ; **MICHLITS H** ; **SCHMIDT D** ; **OBINGER C** ; **FURTMÜLLER PG**. Understanding molecular enzymology of porphyrin-binding α + β barrel proteins - One fold, multiple functions. *Biochim Biophys Acta Proteins Proteom.*, January 2021, vol. 1869 (1), 140536

- **GAO JL** ; **NGUYEN KA** ; **HUNTER N**. Characterization of a hemophore-like protein from Porphyromonas gingivalis. *J Biol Chem.*, 2010, vol. 285 (51), 40028-38
- **WANDERSMAN, C.** ; **DELEPELAIRE, P.** Haemophore functions revisited. *Molecular Microbiology*, 2012, vol. 85, 618-631
- **BATEMAN TJ** ; **SHAH M** ; **HO TP** ; **SHIN HE** ; **PAN C** ; **HARRIS G** ; **FEGAN JE** ; **ISLAM EA** ; **AHN SK** ; **HOODA Y**. A Slam-dependent hemophore contributes to heme acquisition in the bacterial pathogen Acinetobacter baumannii. *Nat Commun.*, 2021, vol. 12 (1), 6270
- **MALMIRCHEGINI GR** ; **SJODT M** ; **SHNITKIND S** ; **SAWAYA MR** ; **ROSINSKI J** ; **NEWTON SM** ; **KLEBBA PE** ; **CLUBB RT**. Novel mechanism of hemin capture by Hbp2, the hemoglobin-binding hemophore from Listeria monocytogenes. *J Biol Chem.*, 2014, vol. 289 (50), 34886-99
- **ZHU Y** ; **LECHARDEUR D** ; **BERNARDET JF** ; **KEROUAULT B** ; **GUÉRIN C** ; **RIGAUDEAU D** ; **NICOLAS P** ; **DUCHAUD E** ; **ROCHAT T**. Two functionally distinct heme/iron transport systems are virulence determinants of the fish pathogen Flavobacterium psychrophilum. *Virulence*, 2022, vol. 13 (1), 1221-1241
- **OWENS CP** ; **CHIM N** ; **GRAVES AB** ; **HARMSTON CA** ; **INIGUEZ A** ; **CONTRERAS H** ; **LIPTAK MD** ; **GOULDING CW**. The Mycobacterium tuberculosis secreted protein Rv0203 transfers heme to membrane proteins MmpL3 and MmpL11. *J Biol Che.*, 2013, vol. 288 (30), 21714-28
- **DARTOIS et al.** *Biochemica et Biophysica Acta*, 1993, vol. 1131, 253-360
- Colour Index. Society of Dyers and Colourists